# EUROPEAN PATENT APPLICATION

(11) **EP 2 017 624 A1**
(43) Date of publication of application: **21.01.2009**
(21) Application number: 07741745.9
(22) Date of filing: 17.04.2007
(51) Int. Cl.: G01N 35/02, B01D 29/00, B01D 35/02, C12N 15/00, G01N 33/48, G01N 37/00

(54) **FILTER-CARRYING MICRO PLATE**

(30) Priority: 20.04.2006 JP 2006116668; 29.11.2006 JP 2006322067
(71) Applicant: Dai Nippon Printing Co., Ltd., Tokyo 162-8001 (JP)
(72) Inventor: UEMATSU, Hiroshi, Tokyo 162-8001 (JP); TOKUNAGA, Satoko, Tokyo 162-8001 (JP); TAKEI, Jiro, Tokyo 162-8001 (JP)
(74) Representative: Siegert, Georg
(86) International application number: PCT/JP2007/058309
(87) International publication number: WO 2007/123100

(57) **Abstract**

There is provided a filter-equipped microplate including: an upper container 41 having openings 50 for injection of a substance to be tested; a top packing 44 and a bottom packing 45 that hold filters 46; a middle container 42 that fits with the upper container 41 and has openings 63 through which a test sample that has passed through the filters 46 runs, and that clamps the top packing 44 and the bottom packing 45 against the upper container 41: and a lower container 43 having reservoirs 80 that retain the test sample, the lower container 43 being held in a freely detachable manner against the middle container 42, wherein the middle container 42 has guide walls 64 that suspend down from the openings 63 and provide downward openings 65 to the bottom end, and the reservoirs 80 in the lower container 43 house the guide walls 64, the reservoirs 80 receiving the test sample supplied from the downward openings 65 of the guide walls 64 through the filters 46, and the lower container 43 having vents 90 at a widening slant 92 at the top, that communicate with the outside of the reservoirs 80.

## Description

### Technical Field

The present invention relates to a filter-equipped microplate. More specifically, it relates to a microplate wherein a test sample obtained by filtering a substance to be tested through a filter is collected in a reservoir, and which incorporates a filter with a structure allowing easy separation of the reservoir. Filter-equipped microplates are widely used in fields such as cell tissue culturing and live cultured tissue assay.

Components that can interfere with detection sensitivity are separated from filter-equipped microplates by the filter in advance, i.e. they are filtered, in order to obtain the desired culturing target or to remove out only components that are useful for examination. This is because inclusion of unwanted components can result in excessive prominence of unintended peaks, making it difficult to identify the desired peak points, or can prevent proper reaction of the desired peak points, which creates concern. The active components separated by such filtering are then supplied to subsequent steps.

With such filter-equipped microplates, therefore, it has been necessary to rapidly and easily separate from the microplate only the reservoir holding the active components separated by the filter, or the container comprising the reservoir, before the physical properties of the components are subsequently altered. It is, therefore, very important to be able to detach only the container comprising the reservoir from the microplate in a rapid and easy manner. The microplate also comprises various other elements, and it is important for these elements to be kept in a clean state and to be capable of rapid and reliable assembly.

### Background Art

Conventional filter-equipped microplates are known that are multilayer grooved integrated devices comprising upper and lower test vessels, a filter and a sensor, wherein the sensor can detect an object collected in the lower test vessel through the filter by a label-free method, in order to eliminate extra steps and cost such as cell staining and fluorescent labeling.

There is also known a microplate with a filter provided with an adhesive-coated support plate that prevents splashing, in order to prevent infiltration of liquid culture medium into other cells by splashing when specific cells are supplied through a filter into a cell containing a liquid culture medium and the microplate is shaken to promote culturing in the culture medium.

However, no attention has been given to developing a filter-equipped microplate having a structure wherein the container section, which has the reservoir retaining only the active components that have been separated by the filter, is easily detachable from the microplate.
[Patent document 1] Japanese Patent Published Translation No. 2006-505278 (JP2006505278-T)
[Patent document 2] Japanese Unexamined Patent Publication HEI No. 4-158779 (JP4158779-A)

### Disclosure of the Invention

### Problems to be Solved by the Invention

The hitherto known microplates are generally constructed with a plurality of elements. Such elements must be rapidly and cleanly assembled, but most efforts have been directed only toward developing the filters in such known microplates, whereas it is still difficult to accomplish rapid and hygienic assembly of the elements composing microplates. Moreover, microplates use very thin and easily breakable filters; therefore, there have been accidents that the filters are often displaced or damaged when the microplates are moved. Filtering of a substance to be tested also requires formation of very fine filter meshes, depending on the object of examination, and the long time required for the substance to be tested to pass through such filters is a problem in such cases. Also, when numerous microplates have been necessary to examine large amounts of specimen, it has been difficult to accomplish satisfactory stacking of a number of microplates.

There are problems: the sample in a reservoir during suction at examination can become mixed with the sample in the adjacent reservoirs, reverse flow of the sample can potentially occur when a large negative pressure is applied during the suction; and the sample being suctioned may flow out of the reservoir.

In order to solve these problems, the invention provides a filter-equipped microplate comprising; an upper container having openings for injection of a substance to be tested; a top packing and a bottom packing holding the filters; a connecting member fitted with the upper container, having openings through which a test sample that has passed through the filters runs and clamping the top packing and the bottom packing against the upper container; and a lower container having reservoirs that retain the test sample, the lower container being held in a freely detachable manner with respect to the connecting member.

Also, in order to solve the aforementioned problems, the invention further provides a filter-equipped microplate comprising: an upper container having openings for injection of a substance to be tested, a top packing and a bottom packing holding filters; a middle container fitted with the upper container, having openings through which a test sample that has passed through the filters runs and clamping the packings against the upper container; and a lower container having reservoirs that retain the test sample, the lower container being held in a freely detachable manner against the middle container,
Wherein the middle container has guide walls that suspend down from the openings and provide downward openings to the bottom end, the reservoirs in the lower container house the guide walls, said reservoirs receiving the test sample supplied from the downward openings of the guide walls through the filters, and the lower container has a vent at a widening slant at the top, that communicate with the outside of the reservoirs.

### Means for Solving the Problems

The aforementioned problems are solved by providing a filter-equippedmicroplate according to the following (1)-(23).
(1) There is provided a filter-equipped microplate 110 comprising: an upper container 111 having openings 120 for injection of a substance to be tested; a top packing 113 and a bottom packing 114 holding the filters 115; a connecting member 112 fitted with the upper container 111, having openings 140 through which a test sample that has passed through the filters 115 runs and clamping the top packing 113 and the bottom packing 114 against the upper container 111; and a lower container 116 having reservoirs 160 that retain the test sample, the lower container 116 being held in a freely detachable manner with respect to the connecting member 112.

(2) There is provided a filter-equipped microplate according to (1), wherein the upper container 111 has an external vertical wall 124 that extends vertically downward at the outer periphery; the external vertical wall 124 has a protrusion 125 that projects outward; the connecting member 112 has a standing wall 135 at the outer periphery that extends vertically upward, the standing wall having a protrusion 136 that projects inward; and the protrusions 125, 136 are engaged, whereby fitting between the upper container and connecting member is achieved by said engagement.

(3) There is provided a filter-equipped microplate according to (1) or (2), wherein the upper container 111 has fitting holes 128, each comprising an upper section 129 and a lower section 130, wherein the upper section has a wide diameter hole and the lower section has a narrow diameter hole, and a step 131 is formed between the upper section and the lower section; the connecting member 112 has a hollow lock pin 143 that extends upward, the lock pin having a widening-diameter section 144 at the top, which widening-diameter section comprises a plurality of grooves that extend in the axial direction; and pressing the lock pin 143 from the lower section 130 of the upper container 111 toward the fitting hole 128 causes the widening-diameter section 144 of the lock pin to move toward the center and reduce in diameter, while further pressing causes the lock pin 143 to move to the upper section 129 so that the widening-diameter section 144 engages with the step 131 of the upper container 111, whereby fitting between the upper container 111 and the connecting member 112 is achieved.

(4) There is provided a filter-equipped microplate according to any one of (1) to (3), wherein the connecting member 112 has a retainer wall section 137 that extends downward and a positioning pin 145, the retainer wall section 137 consisting of an outer wall 138 and a slanted inner wall 139 and being placed at the outer periphery in such a manner as to surround the connecting member 112, and the positioning pin 145 having a conical shape, with a plurality thereof being provided at the inner section of the connecting member 112; the lower container 116 comprises on its outer periphery a peripheral rib 161 with a slanted section 162 and a step section 163, and a positioning pin receiver 164 that forms a conical shape; and the lower container 116 is in airtight contact with the connecting member 112 by pressure welding of the slanted section 162 against the slanted inner wall 139, while the positioning pin 145 and positioning pin receiver 164 are loosely fitted across a prescribed spacing.

(5)There is provided a filter-equipped microplate according to any one of (1) to (4), wherein the filter 115 is fabricated by etching of a silicon wafer, and comprises a center section with through-holes of equal dimensions and an outer peripheral section surrounding the center section, the outer peripheral section being formed to a greater thickness than the center section.

(6) There is provided a filter-equipped microplate according to any one of (1) to (5), wherein a sealing member such as an O-ring is fitted on either the slanted inner wall 139 of the connecting member 112 or the slanted section 162 of the lower container 116, whereby airtight fitting is achieved between them.

(7) There is provided a filter-equipped microplate according to (4), wherein the connecting member 112 and the lower container 116 are fitted in an airtight manner at the slanted inner wall 139 and the slanted section 162 while being fitted loosely at the other sections, and connection of pressure reducing means to one positioning pin receiver 164 allows negative pressure to be produced below the filter, whereby the filtering time can be shortened.

(8) There is provided a filter-equipped microplate according to any one of (1) to (7), wherein the top packing 113 and the bottom packing 114 form an integral structure, thus reducing the number of component parts and facilitating the assembly operation.

(9) There is provided a filter-equipped microplate according to any one of (1) to (8), wherein a mark is provided on the filters 115 or a member in contact with the filters to identify the assembly location, thus allowing more precise positioning of both and permitting automation of the assembly operation while facilitating mass production.

(10) There is provided a filter-equipped microplate 40, 40A comprising: an upper container 41, 41A having openings 50, 50A for injection of a substance to be tested; a top packing 44, 44A and a bottom packing 45, 45A holding filters 46, 46A; a middle container 42, 42A fitted with the upper container 41, 41A, having openings 63 through which a test sample that has passed through the filters 46, 46A runs and clamping the top packing 44, 44A and the bottom packing 45, 45A against the upper container 41, 41A; and a lower container 43, 43A having reservoirs 80, 80A that retain the test sample, the lower container 43, 43A being held in a freely detachable manner against the middle container 42, 42A, wherein the middle container 42, 42A has guide walls 64, 64A that suspend down from the openings 63 and provide downward openings 65 to the bottom end, the reservoirs 80, 80A in the lower container 43, 43A house the guide walls 64, 64A, said reservoirs 80, 80A receiving the test sample supplied from the downward openings 65 of the guide walls 64, 64A through the filters 46, 46A, and the lower container 43, 43A has a vent 90 at a widening slant 92 at the top, that communicates with the outside of each of the reservoirs 80, 80A.

(11) There is provided a filter-equipped microplate according to (10), wherein a plurality of vents 90 are provided.

(12) There is provided a filter-equipped microplate according to (11), wherein two vents 90 are provided.

(13) There is provided a filter-equipped microplate according to any one of (10) to (12), wherein the bottom ends of the guide walls 64, 64A suspend to a depth of at least half of the reservoirs 80, 80A.

(14) There is provided a filter-equipped microplate according to any one of (10) to (13), wherein an auxiliary packing 47, 47A is mounted between the middle container 42, 42A and lower container 43, 43A.

(15) There is a filter-equipped microplate according to (14), wherein the auxiliary packing 47, 47A is bonded to the lower container 43, 43A by different material molding or insert molding.

(16) There is provided a filter-equipped microplate according to any one of (10) to (15), wherein the upper container 41, 41A has an external vertical wall 52, 52A that extends vertically downward at the outer periphery and the external vertical wall 52, 52A has a protrusion 53 that projects outward; the middle container 42, 42A has a standing wall 60, 60A at the outer periphery that extends vertically upward, the standing wall 60, 60A having a protrusion 61 that projects inward; and the protrusions 53, 61 are engaged, whereby fitting between the upper container 41, 41A and middle container 42, 42A is achieved by said engagement.

(17) There is provided a filter-equipped microplate according to any one of (10) to (16), wherein the upper container 41, 41A has a fitting hole 56, each comprising an upper section 57 and a lower section 58, wherein the upper section 57 has a widening-diameter hole and the lower section 58 forms a hole whose diameter narrows from bottom to top, with a step 59 being formed between the upper section 57 and the lower section 58; the middle container 42, 42A has a hollow lock pin 67 that extends upward, the lock pin 67 having a widening-diameter section 69 at the top, which widening-diameter section 69 comprises a space 48 that extends in the axial direction; and pressing the lock pin 67 from the lower section 58 of the upper container 41, 41A toward the fitting hole 56 causes the widening-diameter section 69 of the lock pin 67 to move toward the center and reduce in diameter, while further pressing causes the lock pin 67 to move to the upper section 57 so that the widening-diameter section 69 engages with the step 59 of the upper container 41, 41A, whereby fitting between the upper container 41, 41A and the middle container 42, 42A is achieved.

(18) There is provided a filter-equipped microplate according to any one of (10) to (17), wherein the filters 46, 4 6A are fabricated by etching of a silicon wafer, and each comprises a center section with through-holes of equal dimensions and an outer peripheral section extending from the center section through the slanted section and surrounding the center section, the outer peripheral section being formed to a greater thickness than the center section.

(19) There is provided a filter-equipped microplate according to any one of (10) to (18), wherein each constituent element is composed of a transparent material.

(20) There is provided a filter-equipped microplate according to any one of (10) to (19), wherein the upper container 41A has a flange standing section 86 that extends upward from the outer peripheral section and an open standing section 87 that extends upward continuously from the opening 50A, the flange standing section 86 and the open standing section 87 extending up to essentially the same height.

(21) There is provided a filter-equipped microplate according to any one of (10) to (20), wherein the middle container 42A has a standing wall 60A extending upward from the outer peripheral section and an inner standing wall 88 extending upward from the inside at a prescribed distance from the standing wall 60A, an external vertical wall 52A suspended from the upper container 41A is fitted in the space defined between the standing wall 60A and the inner standing wall 88, and the height of the inner standing wall 88 is lower than the height of the standing wall 60A.

(22) There is provided a filter-equipped microplate according to (21), wherein the inner standing wall 88 holds the outer perimeters of the top packing 44A and the bottom packing 45A.

(23) There is provided a filter-equipped microplate according to any one of (14) to (22), wherein the middle container 42A has a protrusion 89 on the side in contact with the auxiliary packing 47A.

### Effect of the Invention

The filter-equipped microplate of (1) above according to the invention provides a microplate composed of highly simplified elements that can be rapidly assembled, and that is hygienic and allows safe support of fragile filters, while also reliably preventing their displacement.

The filter-equipped microplate of (2) above according to the invention provides a microplate that can be easily assembled and allows a filtered test sample to be easily removed from the microplate, thus eliminating the need for skill for carrying out the procedures. The filter-equipped microplate of (3) above provides a microplate that allows easy and precise connection between the connecting member and the upper container.

The filter-equipped microplate of (4) above according to the invention provides a microplate that allows stable positioning at the resting location. The filter-equipped microplate of (5) above provides a microplate that can be set without destroying thin, fragile filters, and allows the proper positioning to be constantly maintained.

The filter-equipped microplates of (6) and (7) above provide microplates that allow the filtering time to be shortened. The filter-equipped microplates of (8) and (9) above provide microplates that have fewer component parts and thus simplify the assembly procedure and allow mass production.

Thefilter-equipped microplate of (10) above according to the invention provides a microplate composed of highly simplified elements that can be rapidly assembled, and that is hygienic and allows safe support of fragile filters, while also reliably preventing their displacement. In addition, since a vent is provided adjacent to the reservoir, through which the sample is forcibly drawn into the reservoir, an effect of efficient pressure reduction by suction can be expected. As a result, a rapid and reliable filtering operation can be carried out and the initial time required for the culturing or examination procedures can be shortened.

The filter-equipped microplates of (11) and (12) above according to the invention, which have multiple vents, allow the filtering process to be controlled for the optimum time for a given sample. As a result, it is possible to minimize changes in the sample by its contact with air, thus allowing very precise analysis results to be obtained as expected. Also, since the downward openings at the bottom ends of the guide walls extend downward to a point of greater than half the reservoirs in the filter-equipped microplate of (13) above, it is possible to prevent unexpected splashing of the sample supplied from the downward openings into the spaces, thus allowing all of the sample passing through the filters to be used for analysis and thus achieving efficient operation.

The filter-equipped microplates of (14) and (15) above according to the invention have auxiliary packing placed between the middle container and lower container. As a result, constant movement of air through the vent provided in the upper section of the reservoir is ensured. The auxiliary packing can also be placed at the prescribed location using very simple and reliable means. The filter-equipped microplates of (16) and (17) above according to the invention allow simple, rapid and reliable assembly of the upper container and the middle container by a single pressing action. Also, the filter-equipped microplate of (18) above can provide filters with a structure whereby setting of thin, fragile filters can be accomplished without their destruction, so that such filters can consistently be set at the proper position. The filter-equipped microplate of (19) above is composed of transparent materials, and therefore the operator can reliably terminate supply of the sample housed in the reservoir before it reaches the downward opening of the guide wall, thus allowing removal of the air in the reservoir to be achieved consistently and preventing suction of the sample into the pressure reduction apparatus.

Also, the filter-equipped microplate of (20) above according to the invention has a flange standing section 86 extending upward from the outer peripheral section of the upper container 41A and an open standing section 87 extending upward in a continuous manner from the opening 50A, such that they extend to essentially the same height, and therefore the openings through which the sample is provided are enlarged and the height of the microplate as a whole is increased, thus facilitating its handling. According to the filter-equipped microplates of (21) and (22) above, the inner standing wall 88 cooperates with the standing wall 60A to firmly fit and hold the upper container 41A, while the inner standing wall 88 holds the top packing 44A and the bottom packing 45A. This can provide a structurally stable, rigid microplate.

The filter-equippedmicroplateof (23) above according to the invention is provided with a protrusion 89 that keeps the auxiliary packing 47A constantly attached to the lower container 43A when the lower container 43A is removed from the middle container 42A after the sample has been drawn into the reservoirs 80A, thereby preventing the risk of unintentionally contaminating the sample in the reservoir 80A by the auxiliary packing 47A.

### Brief Description of Drawings

Fig. 1 is a plan view of the filter-equipped microplate of Example 1 according to the invention.
Fig. 2 is a cross-sectional view of Fig. 1 along line A-A.
Fig. 3 is a cross-sectional view of Fig. 1 along line B-B.
Fig. 4 is an enlarged view of section X of Fig. 2.
Fig. 5 is a cross-sectional view of Fig. 1 along line C-C.
Fig. 6 is an enlarged view of section Y of Fig. 3.
Fig. 7 is a plan view of the filter-equipped microplate of Example 2 according to the invention.
Fig. 8 is a cross-sectional view of Fig. 7 along line 2-2, showing the location of use.
Fig. 9 is an enlarged view of the section of circle 3 of Fig. 8.
Fig. 10 is a cross-sectional view of Fig. 7 along line 4-4.
Fig. 11 is an enlarged view of the section of circle 5 of Fig. 10.
Fig. 12 is a magnified cross-sectional view of Fig. 7 along line 6-6.
Fig. 13 is a plan view of the upper container of a filter-equipped microplate according to the invention.
Fig. 14 is a cross-sectional view of Fig. 13 along line 8-8.
Fig. 15 is an enlarged view of the section of circle 9 of Fig. 14.
Fig. 16 is a cross-sectional view of Fig. 13 along line 10-10.
Fig. 17 is an enlarged view of the section of circle 11 of Fig. 16.
Fig. 18 is an enlarged view of the section of circle 12 of Fig. 16.
Fig. 19 is a plan view of the middle container of a filter-equipped microplate according to the invention.
Fig. 20 is a back view of the middle container shown in Fig. 19.
Fig. 21 is a side view of Fig. 19 along line 15-15.
Fig. 22 is a cross-sectional view of Fig. 19 along line 16-16.
Fig. 23 is an enlarged view of the section of circle 17 of Fig. 22.
Fig. 24A is a magnified cross-sectional view of the lock pin shown in Fig. 22.
Fig. 24B is a magnified top view of the lock pin shown in Fig. 22.
Fig. 25 is a cross-sectional view of Fig. 19 along line 19-19.
Fig. 26 is a plan view of the lower container of a filter-equipped microplate according to the invention.
Fig. 27 is a back view of the lower container shown in Fig. 26.
Fig. 28 is a side view of Fig. 26 along line 22-22.
Fig. 29 is a cross-sectional view of Fig. 26 along line 23-23.
Fig. 30 is an enlarged view of the section of circle 24 of Fig. 29.
Fig. 31 is a magnified cross-sectional view of Fig. 26 along line 25-25.
Fig. 32 is a cross-sectional view of Fig. 26 along line 26-26.
Fig. 33 is a plan view of the top packing to construct a filter-equipped microplate according to the invention.
Fig. 34 is a back view of the top packing shown in Fig. 33.
Fig. 35 is a cross-sectional view of Fig. 33 along line 29-29.
Fig. 36 is an enlarged view of the section of circle 30 of Fig. 35.
Fig. 37 is a magnified cross-sectional view of the section indicated by the lead 31 in Fig. 33 and Fig. 34.
Fig. 38 is a plan view of the bottom packing to construct a filter-equipped microplate according to the invention.
Fig. 39 is a plan view of the auxiliary packing to construct a filter-equipped microplate according to the invention.
Fig. 40A is a set of enlarged top views (a-e) showing five different embodiments of the filter-equipped microplates according to the invention.
Fig. 40B is a set of enlarged cross-sectional views (A-E) showing five different embodiments of the filter-equipped microplates according to the invention.
Fig. 41 is a view similar to Fig. 9 showing Example 3 according to the invention.
Fig. 42 shows a lower container affixed with markings identifying the position of each opening, according to the invention.
Fig. 43 shows a lower container rib and a lower container guide according to the invention.

### Reference Signs List

30 Lower container rib
35 Lower container guide
41, 41A Upper containers
42, 42A Middle containers
43, 43A Lower containers
44, 44A Top packing
45, 45A Bottom packing
46, 46A Filters
47, 47A Auxiliary packing
48 Space
49 Slanted section
50, 50A Openings
51, 51A Conical shaped walls
52, 52A External vertical walls
53 Protrusion
54, 54A Flanges
55 Contact ring
56 Fitting hole
57 Upper section
58 Lower section
59 Step
60, 60A Standing walls
61 Protrusion
62, 62A Retainer wall sections
63 Opening
64, 64A Guide walls
65 Downward opening
66 Contact ring
67 Lock pin
68 Standing side
69 Widening-diameter section
70, 70A Contact rings
71 Opening
72 Step
73 Hole
74 Indentation
75 Opening
76 Hole
77 Pad section
80, 80A Reservoirs
81 Peripheral rib
82 First suspended section
83 Second suspended section
84 Third suspended section
85 Stack rib
86 Flange standing section
87 Open standing section
88 Inner standing wall
89 Protrusion
90 Vent
91 Top
92 Widening slant
93 Curve
94 Opening
95 Curve
96 Circumscribed circle
97 Circumscribed circle
98 Lengthwise axial line of reservoir
99 Lengthwise axial line of guide wall
L1-L7 Dimensions
110 Filter-equipped microplate
111 Upper container
112 Connecting member
113 Top packing
114 Bottom packing
115 Filter
116 Lower container
120 Opening
121 Prescribed dimension
122 Conical-shaped wall
123 Prescribed dimension
124 External vertical wall
125 Protrusion
126 Flange
127 Contact ring
128 Fitting hole
129 Upper section
130 Lower section
131 Step
135 Standing wall
136 Protrusion
137 Retainer wall section
138 Outer wall
139 Slanted inner wall
140 Opening
141 Guide wall
142 Contact ring
143 Lock pin
144 Widening-diameter section
145 Positioning pin
146 Pedestal
150 Opening
151 Step
155 Opening
160 Reservoir
161 Peripheral rib
162 Slanted section
163 Step section
164 Positioning pin receiver
166 Resting surface
170 Space
171 Passageway

### Best Mode for Carrying Out the Invention

Preferred embodiments of the present invention will now be described.

### Examples

### [Example 1]

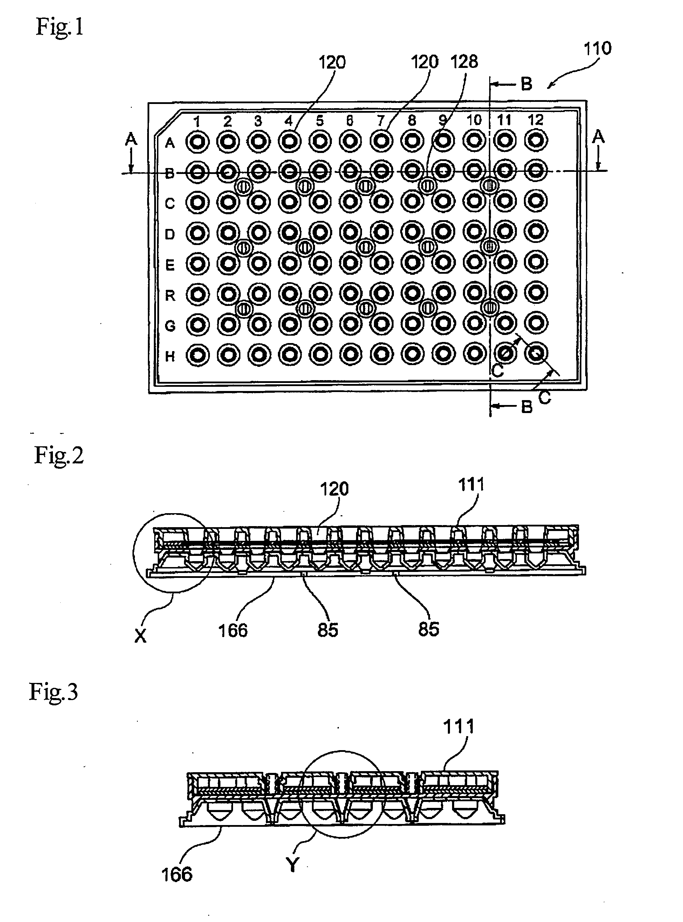

Fig. 1 is an enlarged plan view of a filter-equipped microplate 110 according to one embodiment of the device of the invention. The microplate has a rectangular-shaped surface as shown in the drawing, and an overall cuboid form with approximate dimensions of, for example, long side (120-150 mm) × short side (80-100 mm) × thickness (10-20 mm). However, one skilled in the art will readily appreciate that the dimensions and shape can be varied according to the purpose and requirements. The filter-equipped microplate 110 of the invention may therefore have a surface with a circular or elliptical shape, for example, instead of a rectangular or other quadrilateral shape as shown in the drawing. However, a rectangular shape is assumed in the following example. The microplate 110 has a plurality of openings 120 formed on the front side, i.e. the top surface (a total of 12 × 8 = 96 in Fig. 1), and a culture solution (for example, a substance to be tested such as sampled blood) is supplied into the microplate through the openings 120.

As shown in Figs. 2 to 5, the microplate 110 of the invention has a structure composed of an upper container 111 in which the openings 120 are formed, a connecting member 112 that fits and is engaged with the container 111, a top packing 113 and a bottom packing 114 sandwiched between the upper container 111 and the connecting member 112, filters 115 placed at prescribed location of the top packing 113 which are positioned in contact with the upper container 111, and a lower container 116 placed below the connecting member 112.

As seen in Figs. 2 to 5, the upper container 111 and the connecting member 112 have approximately the same area, and only the lower container 116 has a somewhat larger area than the upper container 111 and the connecting member 112. The packings 113, 114 are placed in and surrounded by the upper container 111. The filters 115 placed in the top packing 113 have a slightly larger area than the openings 120, and in the examples shown in Figs. 2 to 5, a total of 96 filters 115 are placed under the openings 120, in a one-to-one correspondence with the openings 120. The upper container 111, the connecting member 112 and the lower container 116 are formed of a plastic material with some elasticity (for example, polyethylene resin), having stable properties that render them generally resistant to chemical changes. The packings 113, 114 are,formed of a soft material (for example, silicon) having stable properties that render them also resistant to chemical changes. The filters 115 are formed by, for example, etching a silicon wafer.
The packings 113, 114 will be described as two separate parts to facilitate understanding of the construction in the examples that follow, but there is no limitation to these, and it is a simple matter for one skilled in the art to alter the manner of holding the filter and form the packings into an integrally molded unit in order to reduce the number of parts and simplify the assembly procedure.

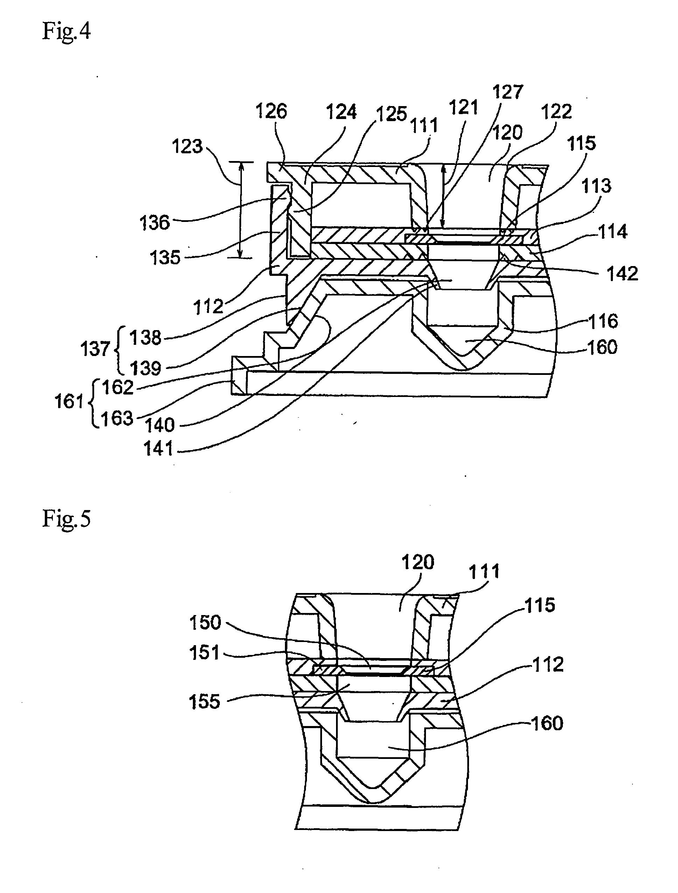

As shown in Figs. 1 to 4, the upper container 111 has the circular openings 120 with the same prescribed area arranged almost regularly across the entire surface. The openings 120 are formed of conical-shaped walls 122 that are depressed in an integral manner by the prescribed dimension 121 in the perpendicular direction, downward from the surface of the upper container 111. Also, as shown in Fig. 4, an external vertical wall 124 extending by the prescribed dimension 123 downward essentially perpendicularly from the surface is also formed in an integrally depressed manner along the entire outer periphery of the surface of the upper container 111. The prescribed dimension 123 of the external vertical wall 124 is slightly greater than the dimension 121 of the conical-shaped walls 122. Due to this difference in dimensions, the external vertical wall 124 surrounds the packings 113, 114 within the region defined by the external vertical wall 124. Also, an outward protrusion 125 with a roughly circular cross-section is formed on the outer side of the external vertical wall 124. The protrusion 125 is preferably formed across the entire outer side of the external vertical wall 124, but there is no limitation to this construction.
On the outside of the external vertical wall 124 of the upper container 111 there is formed a flange 126 oriented outward in the radial direction, and the flange 126 has the function of protecting the connecting member 112 described hereunder. Also provided are contact rings 127 (two in the example shown in Fig. 4) forming circles that are roughly concentric with the surface under each conical-shaped wall 122, which is in contact with the top packing 113. The rings 127 perform the function of pressing against the packing 113 to prevent slippage of the packing.

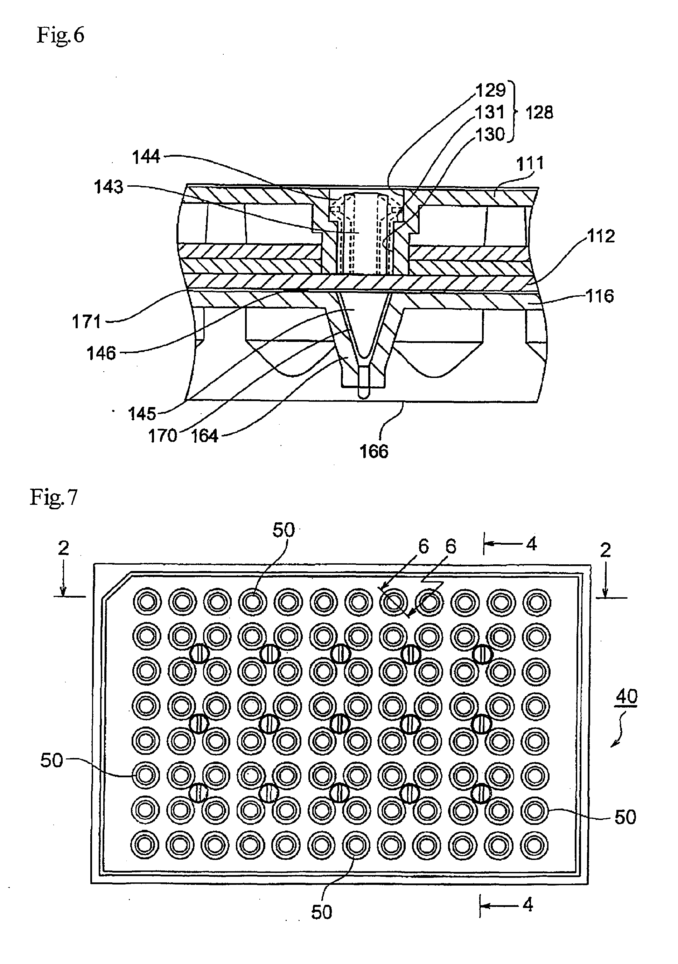

As shown in Fig. 1, Fig. 3 and Fig. 6, the upper container 111 also has a plurality of fitting holes 128 in the spaces between the openings 120. In the example shown in Fig. 1, the fitting holes 128 are provided in the spaces between the 2nd and 3rd rows, between the 4th and 5th rows, between the 6th and 7th rows, between the 8th and 9th rows and between the 10th and 11th rows from the right in the longitudinal direction, and in the spaces between the 2nd and 3rd rows, between the 4th and 5th rows and between the 6th and 7th rows from the top in the transverse direction, for a total of 15 holes, but there is no limitation to this construction. More or fewer holes may be used.
As shown in Fig. 6, the fitting hole 128 is formed as hole with a circular cross-section and a fixed diameter, suspended from the surface of the upper container 111. Each hole comprises an upper section 129 with a somewhat smaller diameter than the diameter of the opening 120, and a lower section 130 having a diameter that is reduced in size compared to the upper section. A step 131 is formed between the upper section 129 and lower section 130.
Small thickened sections are formed on the outer perimeter of the surface of the upper container 111 and around the opening 120 for reinforcement, as shown in Fig. 4. These protruding sections do not need to be formed at the lower sections of all of the fitting holes 128, and for example, they may be in the spaces between the 4th and 5th rows and between the 8th and 9th rows from the right in the longitudinal direction and between the 2nd and 3rd rows and between the 6th and 7th rows from the top in the transverse direction, for a total of 4. However, there is no limitation to this construction and more or fewer protruding sections may be used.

The connecting member 112 is placed opposing the upper container 111, and provides the function of clamping the packings 113, 114 in cooperation with the upper container. It also provides the function of connecting the upper container 111 and the lower container 116. The connecting member 112 has a standing wall 135 that rises in an integral fashion from its outer periphery, roughly in the perpendicular direction toward the upper container. As shown in Fig. 4, the standing wall 135 rises to a position that surrounds the outside of the external vertical wall 124 of the upper container 111. An inward pointing protrusion 136 with, for example, a circular cross-section, is integrally formed in the inner side of the standing wall 135.
The protrusion 136 is formed at a position between a protrusion 125 formed in the external vertical wall 124 of the upper container 111 and the flange 126 of the upper container 111, so that it engages with the protrusion 125. This allows the protrusion 125 of the upper container 111 and the protrusion 136 of the connecting member 112 to fit together for integration of both members as a unit. The protrusion 136 is preferably formed across the entire inner side of the standing wall 135, but there is no limitation to this construction. That is, it may be formed only at the position at which the protrusion 125 of the upper container 111, with which it fits, is formed. This will simplify the fitting operation and allow more optimal use of the materials. The standing wall 135 extends upward from the position of the protrusion 136 to a point that does not contact the flange 126 of the upper container 111. This will reinforce the standing wall 135 while stabilizing the fit between the upper container 111 and the connecting member 112.

A retainer wall section 137 is also integrally formed near the outer periphery of the connecting member 112, suspending downward therefrom in the direction opposite from the standing wall 135. The retainer wall section 137 is preferably suspended in such a manner as to surround the entire outer periphery of the connecting member 112. The retainer wall section 137 preferably has a triangular cross-section and consists of an outer wall 138 that suspends approximately perpendicularly from the connecting member 112, and also a slanted inner wall 139 running obliquely inward from the bottom end of the wall.
The connecting member 112 has a plurality of openings 140 with circular cross-sections in the same number as the openings 120 formed in the upper container 111 (96 openings in Fig. 4), which are positioned to correspond to the openings 120 when the connecting member 112 is assembled with the upper container 111. As shown in Fig. 4, the opening 140 has a slightly smaller diameter than the opening 120. Each opening 140 consists of a thin valve-like guide wall 141 suspending downward integrally from the connecting member 112 toward the direction of the center of the opening 140. The lower edge of the guide wall 141 suspending downward toward the direction of the center of the opening 140 forms the opening 140. The edge of the guide wall 141 extends in the direction of a reservoir 160 of the lower container 116 described below, and has the function of serving as a reliable guide into the reservoir 160, for specimens such as culture solution which are provided to the opening 120 of the upper container 111.
At the top of the connecting member 112 at the section where the guide wall 141 is formed, there are formed a plurality (2 in Fig. 4) of contact rings 142 around and in a roughly concentric manner with the guide wall 141. The rings 142 contact the bottom packing 114 and perform the function of pressing against the packing 114 to preventing slippage of the packing.

As also shown in Figs. 1, 3 and 6, the connecting member 112 has a plurality (15 in these drawings) of lock pins 143 standing up from the top of the connecting member 112 at locations corresponding to the fitting holes 128 of the upper container 111, and they are integrally formed at those locations. The lock pins 143 preferably have hollow shapes. The top of each lock pin 143 has a widening-diameter section 144 with widening dimensions, and the apex of the widening-diameter section 144 has a shape resembling an abacus bead. Grooves of a prescribed width are provided every 90 degrees, for example, running in the longitudinal direction, from the top of the abacus bead-shaped section across the hollow standing section. This produces an elastic property allowing expansion and contraction in the radial direction at the top of the abacus bead-shaped section.
The grooves may be formed at other angles, such as 60 degree angles or 120 angles. It is important, however, for the grooves to be formed at equivalent angles, so that the elastic property is not exhibited in an asymmetrical manner. The lock pin 143 has a length such that it does not protrude from the surface of the upper container 111 when the pin is fitted in the upper container 111. This will allow stable stacking when a plurality of microplates of the invention are stacked. In the example shown in Fig. 6, the lock pin 143 cannot be separated from the fitting hole 128 since it is in the "fixed" state; however, when a problem has been found in the filter after assembly, for example, it may be necessary to remove and exchange the defective filter. Thus, the widths of the longitudinal grooves formed in the widening-diameter section 144 of the lock pin 143 can be appropriately adjusted so that it can be separated from the fitting hole 128 when necessary.

A plurality (15 in Fig. 6) of positioning pins 145 are also formed, preferably suspended in an integral manner, from the bottom of the connecting member 112 at locations corresponding to the locations of the lock pins 143 of the connecting member 112. Each positioning pin 145 has a roughly conical shape that narrows downward. The positioning pins 145 perform the function of locating the fitting positions for the lower container 116 described below. A pedestal 146 is also formed concentrically with each positioning pin 145, preferably in an integral manner with the connecting member. The pedestals 146 function as spacing members to ensure a prescribed spacing between the connecting member 112 and lower container 116.

Two packing members are sandwiched between the upper container 111 and connecting member 112. They are a top packing 113 held by the upper container 111 and a bottom packing 114 held by the connecting member 112. The packings are formed to essentially the same surface area with essentially the same material which may be, for example, a soft material such as silicon, and when the packings 113, 114 are pressed against the upper container 111 and the connecting member 112, respectively, contact rings 127, 142 formed in the upper container 111 and the connecting member 112, respectively, sink into the packings of soft material, thus preventing slippage of the packings 113, 114 while also preventing displacement between the packings. As mentioned above, the outer perimeters of the packings are held by and in contact with the inside of the external vertical wall 124 of the upper container 111, for positioning of the packings.

The top packing 113 has a plurality (96 in Fig. 1) of circular openings 150 formed at positions corresponding to the openings 120 of the upper container 111. A step 151 is provided below the packing 113 around the periphery of each opening 150. The shape and depth of the step 151 matches the shape and thickness of the filter 115. The diameter of the opening 150 is essentially equal to the diameter defined by the bottom end of the conical-shaped wall 122 formed in the upper container 111.

The bottom packing 114 likewise has a plurality (96 in Fig. 1) of circular openings 155 formed at positions corresponding to the openings 120 of the upper container 111. The diameter of the opening 155 is also preferably essentially equal to the diameter defined by the bottom end of the conical-shaped wall 122 formed in the upper container 111. Thus, the bottom packing 114 has the same dimensions and shape as the top packing 113 but does not have the steps 151 that are formed in the top packing 113. The bottom packing 114 also has essentially the same thickness as the top packing 113. In the examples shown in Fig. 5, the steps 151 for holding of the filter 115 are formed in the top packing 113 for easier workability during assembly, but the steps 151 may instead be formed in the bottom packing 114. Also, steps having dimensions with approximately half the thickness of the outer perimeter of the filter 115 may also be formed in the top packing and bottom packing.

A plurality (96 in Fig. 1) of reservoirs 160 are formed in the lower container 116, suspending roughly perpendicular from the surface of the lower container 116, at locations corresponding to the openings 120 of the upper container 111 (see Fig. 4 and Fig. 5). The reservoirs 160 serve the function of receiving and housing only the filtered test sample after the substance to be tested supplied through the openings 120 of the upper container 111 has been filtered by the filter 115. Each reservoir 160 has a large enough volume to house the necessary amount of test sample.
A peripheral rib 161 is formed in the outer peripheral section of the lower container 116, extending downward from the reservoir 160. The peripheral rib 161 comprises a slanted section 162 extending out downward from the surface of the lower container 116, and a step section 163 extending outward in a step-like fashion from the bottom end of the slanted section 162. The peripheral rib 161 allows stacking to be carried out in a stable manner when a plurality of microplates 110 according to the invention are stacked. The bottom end of the peripheral rib 161 is therefore located lower than the reservoir 160. The slanted section 162 is formed at a slant with an angle so that it essentially contacts the slanted inner wall 139 of the connecting member 112 in a firm manner. This allows the lower container 116 and connecting member 112 to be firmly fitted.

Also, as shown in Fig. 6, a plurality (15 in this drawing) ofpositioningpin receivers 164 are formed, preferably integrally, in the lower container 116. The positioning pin receiver 164 has a conical shape, and the positioning pin 145 of the connecting member 112 is received within it with a prescribed gap. The bottom end of each positioning pin receiver 164 is open.

The filters 115 function to separate out only the necessary elements from the substance to be tested provided from the upper container 111 to each opening 120 and send them to the reservoirs 160 of the lower container 116, and they will normally be made of filtration materials with homogeneous through-holes to precisely collect the specific substances of interest. They will usually be formed by etching of a silicon wafer. The diameters of the holes of the filters 115 are determined according to the size of the specific substance of interest. In order to shorten the filtering time, the filters 115 are thin-films with very small thicknesses. Since they will therefore be prone to damage, the utmost care is necessary for their handling. However, as shown in Fig. 4 and Fig. 5, the sections held in the step 151 of the top packing 113, i.e. the sections where they are set on the microplate, are thicker. Consequently, each filter 115 forms a gradual slant from the thick perimeter section held at the step 151 toward the thin-film section at the center. In the example shown in Fig. 5, the filter 115 has a rectangular shape while the step 151 of the top packing 113 which receives the filter is also rectangular, but there is no limitation to this shape, and the filter 115 and step 151 may be circular, oval or other shapes.

Assembly of the filter-equipped microplate of the invention is accomplished by first setting the upper container 111 upside down. The top packing 113 is then placed on the inverted upper container 111. At this time care must be paid that the step 151 of the top packing 113 is directed upward. The filters 15 are then set on the step 151 of the top packing 113. Here, the filters 115 are placed in the opposite position (inverted) from the position in which they are used during operation. The bottom packing 114 is then placed on top of the top packing 113 and the filters 115. When steps are formed on the bottom packing to hold the filters 115, the bottom packing already having the filters 115 set on the steps is placed on the top packing after the top packing has been set. The top packing 113 and the bottom packing 114 are appropriately placed inside the external vertical wall 124 of the upper container 111.

The connecting member 112 is then set on the bottom packing 114. Here, the connecting member 112 is positioned with the protrusion 136-containing standing wall 135 facing downward so as to cover the bottom packing 114, and the connecting member 112 is pressed toward the upper container 111 until the protrusion 136 of the connecting member 112 fully fits the protrusion 125 formed in the external vertical wall 124 of the upper container 111, thus confirming that the connecting member 112 and the upper container 111 have achieved a reliable fit at the outer perimeter. The 115 lock pins 143 formed in the connecting member 112 are then snapped into the fitting holes 128 of the upper container 111. The diameter dimension of the lock pin 143 is larger than the diameter dimension of the lower section 130 of the fitting hole 128. However, forcible pressing of the lock pins 143 into the lower section 130 causes the abacus bead-shaped tops of the lock pins 143 to contract toward the center by the grooves, thus allowing the lock pins 143 to be easily fitted into the lower section 130.
Further pressing of the lock pins 143 into the fitting holes 128 causes the lock pins 143 to reach the upper sections 130 that have wider dimensions. The tops of the lock pins 143 that have been contracted up to this point are thus restored to their normal diameter states. Consequently, the lock pins 143 are supported at the steps 131 of the fitting holes 128 so that their exit is prevented. It is, therefore, necessary to confirm that the lock pins 143 have reliably engaged with the steps 131. This can be easily confirmed by the sound of the lock pins engaging with the steps when the lock pins move up and down in the axial direction.

When the lock pins 143 and the protrusion 136 provided in the standing wall 135 on the perimeter of the connecting member 112 have been fully snapped into place, assembly of the upper container 111, the connecting member 112, the top packing 113, the bottom packing 114 and the filters 115 is complete. In this state, the contact rings 127 provided under the conical-shaped walls 122 of the upper container 111 are firmly in contact with the top packing 113, while the contact rings 142 provided around the openings of the connecting member 112 are firmly in contact with the bottom packing 114. The packings 113, 114 are both composed of a soft material such as silicon, and are pressure welded together. Slippage of the filters 115 is thus completely prevented. Also, the upper container 111 and the connecting member 112 are bonded into a firm fit by the undercut fit of the protrusions 125, 136 placed around each perimeter, and by the ratchet fit between the fitting hole 128 and the lock pin 143.

Finally, the lower container 116 is attached. The lower container 116 is placed, also upside down, against the connecting member 112 which is inverted with its retainer wall section 137 facing upward. The slanted section 162 of the lower container 116 is placed in firm contact with the slanted inner wall 139 of the connecting member 112. Depending on the shaped angle and the material used, it may also be necessary to consider forming an O-ring groove either in the slanted inner wall 139 of the connecting member 112 or the slanted section 162 of the lower container 116, and setting a sealing member such as an O-ring therein, for setting of the lower container 116, in order to prevent subsequent outflow of air through that section.

Each positioning pin receiver 164 of the lower container 116 is then positioned at the location of each lock pin 143 of the connecting member 112, and inserted therein. The positioning pin receiver 164 of the lower container 116 is set leaving a narrow conical space 170 around the locating pin 145 of the connecting member 112. The reservoir 160 of the lower container 116 is placed so as to be aligned with the opening 140 formed by the guide walls 141 of the connecting member 112. As shown in Fig. 4 and Fig. 6, a slight gap is formed between the lower container 116 and the connecting member 112, and serves as a passageway 171. Thus, fitting is accomplished in an airtight state due to the frictional contact between the connecting member 112 and the lower container 116 at the slanted inner wall 139 and the slanted section 162 formed on their respective outer perimeters, and due to the aid of the O-rings, while slight gaps 170 and a passageway 171 are defined between them at areas other than the slanted surfaces. Pedestals 146 are also formed in the connecting member 112 in order to maintain the passageway 171, and contact of the lower container 116 with the pedestals 146 prevents the passageway 171 from become crushed. This completes assembly of the filter-equipped microplate 110.
The completely assembled microplate is restored to its normal position as shown in Fig. 2, and microplates are stacked for storage. When stacked, the flange 126 formed in the upper container 111 of the lower microplate is set against the stack rib 85 inside the step section 163 of the upper microplate, so that the microplates are held in a reliable manner. Each microplate is therefore stacked in order in a stable posture.
In order to allow mass production of the microplate of the invention, positioning marks may be provided on each part for reference by the assembly device to determine the location of each part and the proper positioning of each part. The assembly operation may then be automated and mass production becomes possible. Since positioning of the filters 115, which are prone to damage against the upper container 111, is important during the operation for assembly of the microplate of the invention, it is important to provide marks identifying the assembly position at the corner section of each filter 115 itself or on the member that is to receive each filter (such as the corner section of the packing), in order to allow proper positioning of the filters 115.

A method of using the filter-equipped microplate 110 of Example 1 according to the invention will now be described. As shown in Fig. 2, one filter-equipped microplate 110 is placed in a prescribed horizontal position. The substance to be tested is then supplied to the openings 120 of the upper container 111 using an appropriate tool such as a pipette. In the microplate 110 shown here, the number of specimens that can be simultaneously supplied as test samples is 96, i.e. the number of openings 120. Referring to Fig. 4 which shows an enlarged view of the cross-section of an opening 120, the substance to be tested supplied into the opening 120 defined by the conical-shaped wall 122 of the upper container 111 is dropped onto the thin filtration surface at the center of the filter 115 which is held against the step 151 of the top packing 113 and sandwiched by the bottom packing 114. Only the portion of the test sample having the prescribed properties can pass through the filter 115. The selectively separated test sample is guided by the guide wall 141 of the connecting member 112 and is retained in the reservoir 160 of the lower container 116.

After a fixed amount of test sample has been retained, only the lower container 116 of the microplate 110 is gently separated from the connecting member 112. This separating procedure allows easy separation by gripping the peripheral rib 161 of the lower container 116 and the retainer wall section 137 of the connecting member 112 and detaching in the vertical direction. While taking care that the test sample retained in the reservoir 160 of the separated lower container does not spill out, the test sample is carried to an examining table in the next step, where the detailed examination of the sample begins.

Depending on the mesh dimensions of the filter 115 used for the invention, the substance to be tested may not easily pass through the filter 115 or the substance to be tested may undergo physical changes during this time. A negative pressure is produced in the reservoir 160 of the lower container 116 in the microplate 110 of the invention in order to allow the substance to be tested retained in the opening 120 to rapidly pass through the filter 115 so that the filtering procedure can be accomplished as rapidly as possible for the substance to be tested, and for this purpose means are provided to forcibly move the substance to be tested through the filter.
Specifically, the bottom end of the positioning pin receiver 164 of the lower container 116 in the microplate which is horizontally supported remains open, and pressure reducing means is connected in an airtight manner to a plurality of suction points (not shown) communicating therewith. The pressure reducing means is then activated to begin pressure reduction. As a result, the air in the reservoir 160 below the filter 115 is evacuated through the passageway 171 and the gap 170 defined between the connecting member 112 and the lower container 116. The pressure in the reservoir 160 is negative pressure, and therefore the substance to be tested is forcibly drawn into the reservoir 160 through the filter 115. As a result, the desired substance in the substance to be tested passes rapidly through the filter 115 to be retained in the reservoir 160. A guide wall 141 is provided in the connecting member 112 so that the test sample that has passed through the filter 115 is not drawn into the passageway. The guide wall 141 guides the test sample that has passed through the filter 115 into the reservoir 160, while also functioning to reduce the effects of the air stream on the test sample so that the test sample is not drawn into the passageway.

### [Example 2]

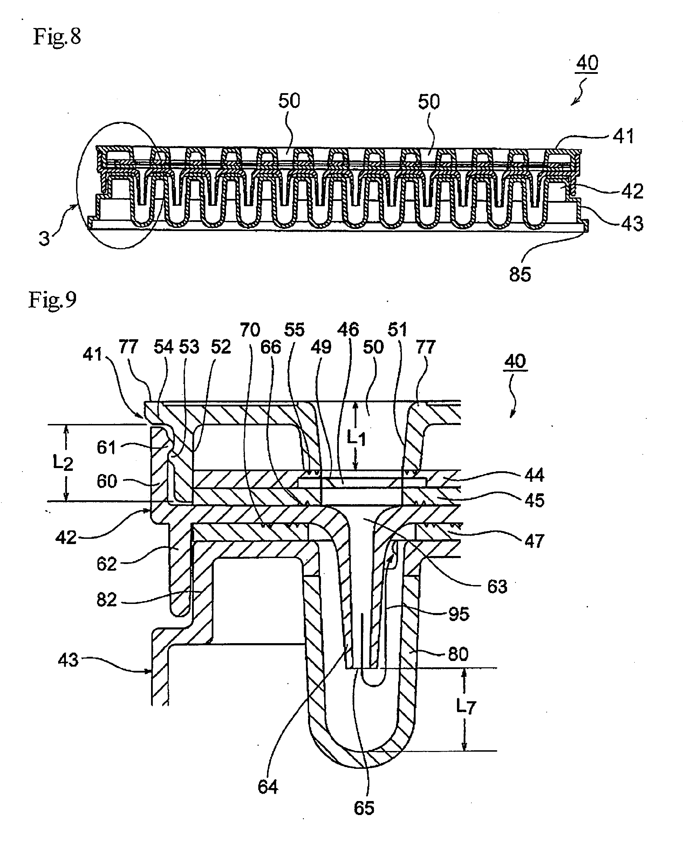

Fig. 7 is a plan view of a filter-equipped microplate 40 according to the invention. The microplate 40 has a rectangular-shaped surface as shown in the drawing, and an overall cuboid form with approximate dimensions of, for example, long side (120-150 mm) × short side (80-100 mm) × thickness (10-30 mm). However, one skilled in the art will readily understand that the dimensions and shape can be varied according to the purpose and requirements. The filter-equipped microplate 40 of the invention may, therefore, have a surface with a circular or elliptical shape, for example, instead of a rectangular or other quadrilateral shape as shown in Fig. 7. However, a rectangular shape is assumed in the following description. The microplate 40 has a plurality of openings 50 formed on the front side, i.e. the top surface (a total of 12 × 8 = 96 in Fig. 7), and a culture solution (for example, a substance to be tested such as sampled blood) is supplied into the microplate 40 through the openings 50.
The structure of the microplate 40 of the invention will now be explained in detail with reference to Figs. 8 to 12. As shown in Fig. 9, the microplate 40 of the invention is composed of an upper container 41 in which the openings 50 are formed, a middle container 42 fitted and engaged with the periphery of the upper container 41, a lower container 43 placed below the middle container 42, a top packing 44 and a bottom packing 45 sandwiched between the upper container 41 and the middle container 42, filters 46 placed at prescribed positions of the top packing 44 which is positioned in contact with the upper container 41, and an auxiliary packing 47 placed between the middle container 42 and the lower container 43.

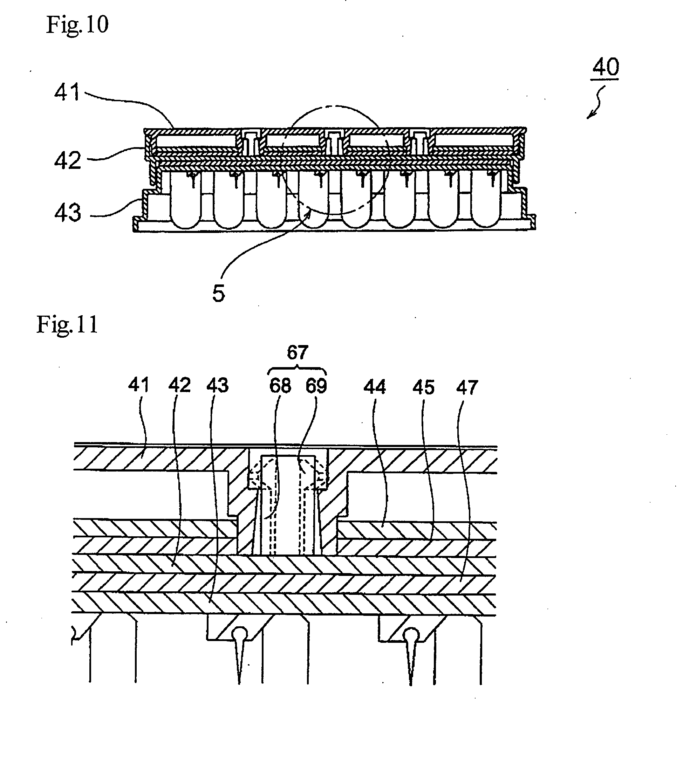

As seen in Figs. 8 and 10, the upper container 41 and the middle container 42 have approximately the same cross-sectional area, and only the lower container 43 has a somewhat larger cross-sectional area than these containers. As seen in Fig. 9, the top packing 44 and the bottom packing 45 are placed in a manner surrounded by the upper container 41. The filter 46 placed in the top packing 44 has approximately the same cross-sectional area as that of the opening 50, and in the drawing, a total of 96 filters 46 are placed under the openings 50, in a one-to-one correspondence with the openings 50. The auxiliary packing 47 is placed in and surrounded by the middle container 42. The upper container 41, middle container 42 and lower container 43 are formed of a plastic material (for example, polypropylene resin) with chemically stable properties and elasticity.

The packings 44, 45, 47 are formed of a soft material (for example, silicon) likewise having chemically stable properties. The containers and packings are all preferably composed of transparent materials. The filters 46 may be formed by, for example, etching a silicon wafer. For easier understanding of the construction, the packings 44, 45, 47 are described below as three separate parts, but there is no limitation to such a construction, and for example, the auxiliary packing 47 may be bonded to the top section of the lower container 43 beforehand by different material molding or insert molding, thereby facilitating the assembly operation. The manner in which the filters are held may be changed to form a unit in which the packings 44, 45 are integrally formed, thus reducing the number of parts and simplifying the assembly operation.

Each of the constituent elements forming the filter-equipped microplate 40 will now be explained in order.

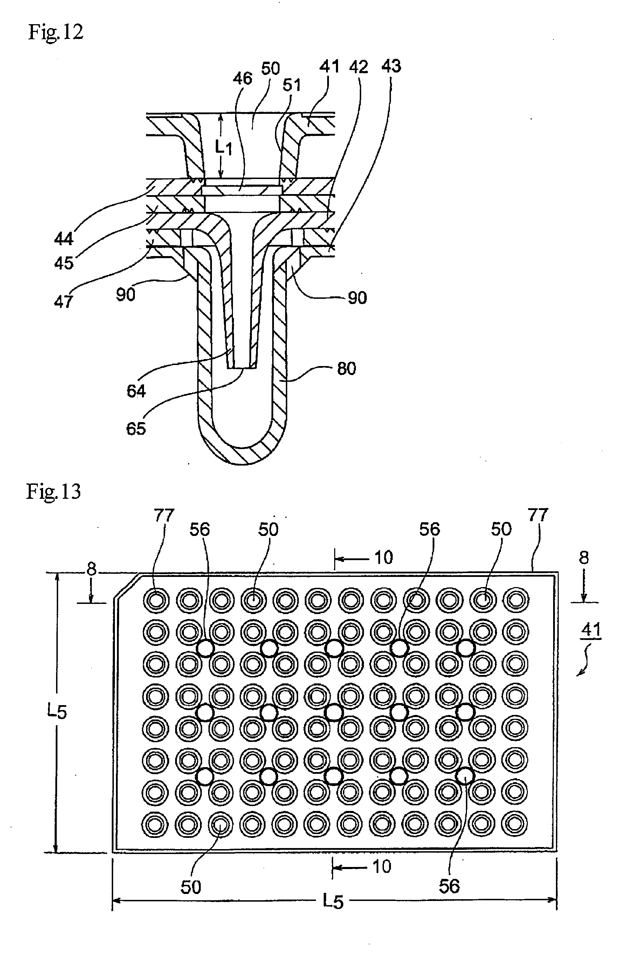

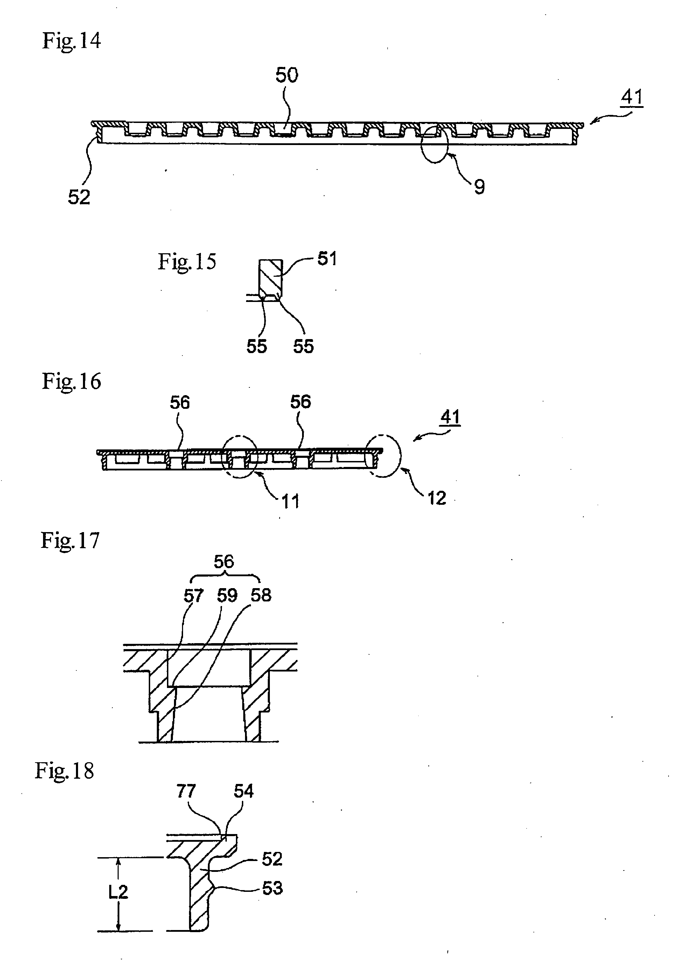

The upper container 41 shown in Figs. 13 to 18 has circular openings 50 with the same prescribed cross-sectional area arranged regularly across the entire surface (Figs. 7-9, Figs. 12-14). As clearly shown in Fig. 6 and Fig. 12, the opening 50 is formed of the conical-shaped wall 51 that is depressed in an integral manner by the prescribed dimension L1 (Fig. 9) in the perpendicular direction, from the surface of the upper container 41 toward the rear side. Also, as shown in Figs. 8-10, Fig. 14 and Fig. 16, an external vertical wall 52 (Fig. 9, Fig. 18) extending by the prescribed dimension L2 (Fig. 9) downward essentially perpendicularly from the surface is also formed integrally along the entire outer periphery of the surface of the upper container 41. Also, an outward protrusion 53 with a roughly circular cross-section, for example, is formed on the outer side of the external vertical wall 52 (Fig. 9, Fig. 18). The protrusion 53 is preferably formed across the entire outer side of the external vertical wall 52, but there is no limitation to this construction, and it may be formed intermittently around the outer side.

On the outside of the external vertical wall 52 of the upper container 41 there is formed, as shown in Fig. 9, a flange 54 oriented outward in the radial direction, and the flange 54 has the function of protecting the middle container 42. Also provided are downward facing contact rings 55 (two in the drawing) (Fig. 9 and Fig. 15) forming circles that are roughly concentric with the surface under each conical-shaped wall 51, which is in contact with the top packing 44. The contact rings 55 contact the top surface of the top packing 44 and perform the function of pressing against the top packing to prevent slippage of the packing.

As shown in Fig. 13, Fig. 16 and Fig. 17, the upper container 41 also has a plurality of fitting holes 56 formed in the empty spaces between the openings 50. In the example shown in Fig. 13, the fitting holes 56 are provided in the spaces between the 2nd and 3rd rows, between the 4th and 5th rows, between the 6th and 7th rows, between the 8th and 9th rows and between the 10th and 11th rows in the longitudinal direction, and in the spaces between the 2nd and 3rd rows, between the 4th and 5th rows and between the 6th and 7th rows in the transverse direction, for a total of 15 holes, but there is no limitation to this construction. More or fewer holes may be used. As shown in Fig. 17, the fitting holes 56 are formed as holes with a circular cross-section and a fixed diameter, suspended from the surface of the upper container 41. Each hole comprises an upper section 57 with a somewhat smaller diameter than the diameter of the openings 50, a lower section 58 having a diameter that is reduced in size compared to the upper section and that widens downward, and a step 59 running in the horizontal direction between the upper section 57 and the lower section 58. The widening of the lower section 58 downward is to facilitate insertion of the lock pin 67 into the fitting hole 56. A small pad section 77 is formed on the outer perimeter of the surface of the upper container 41 and around the opening 50 for reinforcement, as shown in Fig. 9, Fig. 13 and Fig. 18.

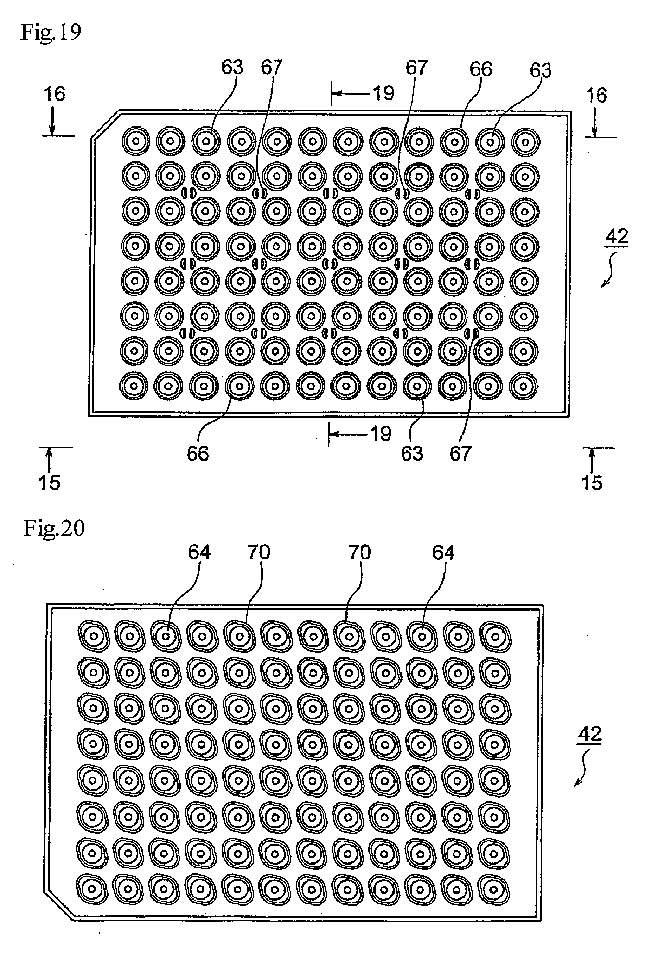

The middle container 42 illustrated in Figs. 19 to 25 is placed opposing the bottom of the upper container 41, and as clearly shown in Figs. 8 to 12, it cooperates with the upper container 41 to clamp the top packing 44 and the bottom packing 45. The middle container 42 also has the function of connecting the upper container 41 and the lower container 43 together. The middle container 42, as clearly shown in Fig. 9, Fig. 22 and Fig. 25, has a standing wall 60 that rises in an integral fashion from its outer periphery, roughly in the perpendicular direction toward the upper container 41. As shown in Fig. 9, the standing wall 60 rises to a position that surrounds the outside of the external vertical wall 52 of the upper container 41. An inward pointing protrusion 61 with, for example, an essentially circular cross-section, is integrally formed in the inner side of the standing wall 60. The inward pointing protrusion 61 is formed at a position between an outward pointing protrusion 53 formed in the external vertical wall 52 of the upper container 41 and the flange 54 of the upper container 41, so that it engages with the upper section of the outward pointing protrusion 53.
Fitting between the outward protrusion 53 of the upper container 41 and the inward protrusion 61 of the middle container 42 in this manner allows close fitting engagement between the two containers. The protrusion 61 is preferably formed across the entire inner side of the standing wall 60, but there is no limitation to this construction. That is, it may be formed only at the position at which the protrusion 53 of the upper container 41, with which it fits, is formed. This will simplify the fitting operation between the upper container 41 and the middle container 42 and allow more economical use of the materials. The standing wall 60 extends upward from the position of the protrusion 61 to a point that does not contact the flange 54 of the upper container 41. This will reinforce the standing wall 60 while stabilizing the fit between the upper container 41 and the middle container 42.

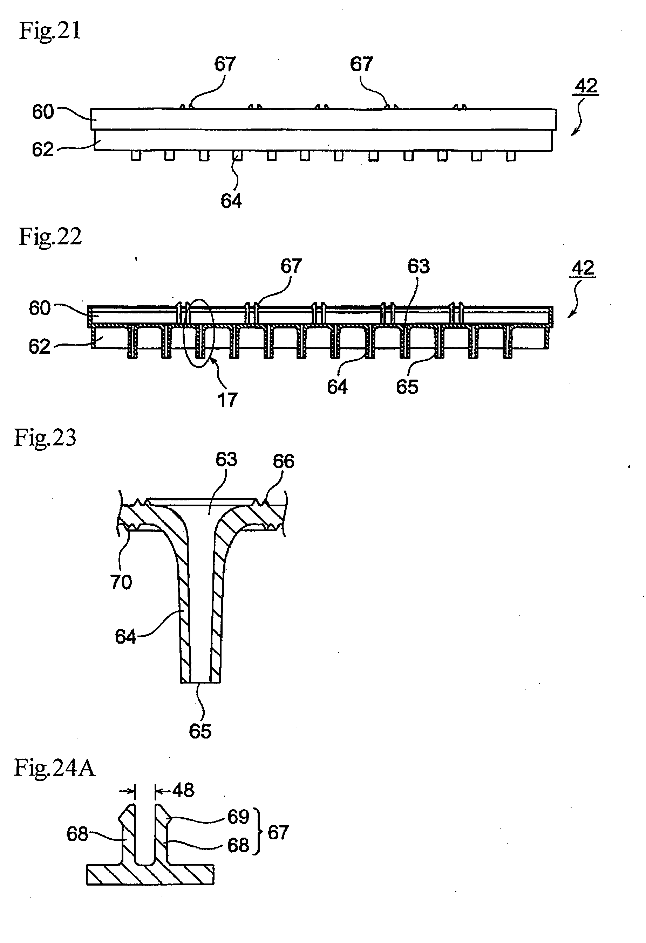

Also, as shown in Fig. 9, a retainer wall section 62 is integrally formed near the outer periphery of the middle container 42, suspending downward therefrom in the direction opposite from the standing wall 60. The retainer wall section 62 is preferably suspended in such a manner as to surround the entire outer periphery of the middle container 42. As shown in Fig. 9, the downward extending retainer wall section 62 preferably extends further downward than the inner section of the standing wall 60 that extends upward. This is a requirement for molding of the middle container 42 rather than for its function. The middle container 42 has a plurality of openings 63 with circular cross-sections in the same number as the openings 50 formed in the upper container 41 (96 openings in the example of the drawings), which are positioned to correspond to the openings 50 when the middle container 42 is assembled with the upper container 41 (Fig. 9, Fig. 19 and Fig. 23).
As shown in Fig. 9, the openings 63 have somewhat smaller diameters than the openings 50 formed in the upper container 41. Each opening 63 consists of a thin valve-like guide wall 64 suspending downward integrally from the middle container 42 toward the direction of the center of the opening 63. The lower edge of the guide wall 64 provides a circular downward opening 65. The edge of the guide wall 64 extends in the direction of the reservoir 80 of the lower container 43 at least to a lower position than the middle section of the reservoir 80, and thus functions to reliably guide the specimen, such as culture solution, supplied to the opening 50 of the upper container 41, to the reservoir 80 while also preventing its splashing against the upper wall surface of the reservoir 80, and preventing reverse flow of the specimen retained in the reservoir 80. A plurality (2 in the example of the drawing) of upward facing contact rings 66 (Fig. 23) are formed, on top of the middle container 42 and at the periphery where each opening 63 is formed, as circles that are approximately concentric with each opening 63. The contact rings 66 contact the bottom side of the bottom packing 45 and perform the function of pressing against the packing 45 to preventing slippage of the packing 45.

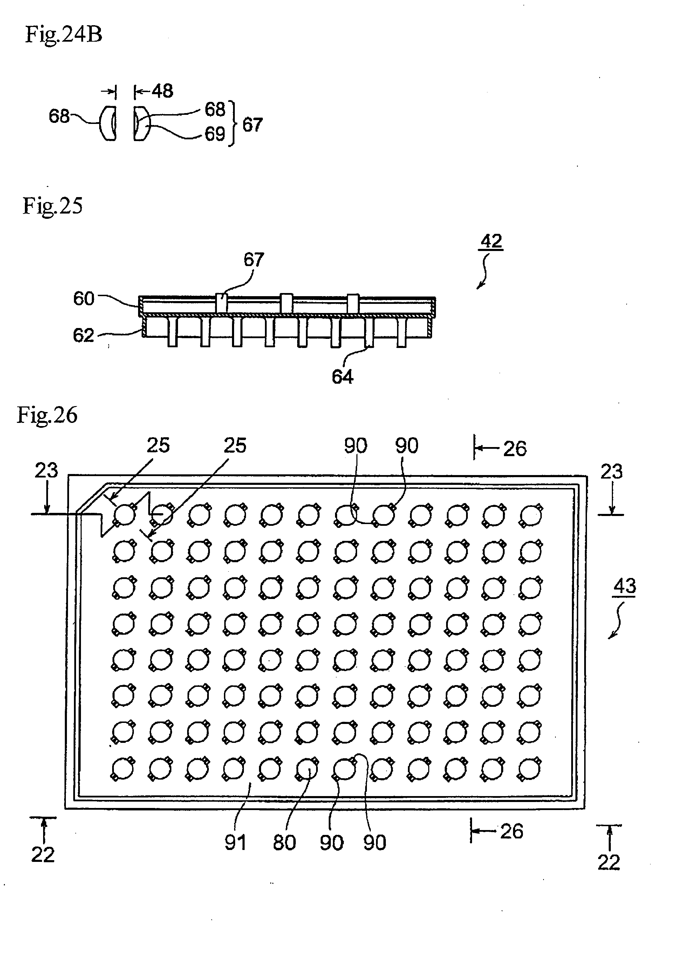

As also shown in Figs. 11, 22, 24A and 24B, the middle container 42 has a plurality (15 in the examples of the drawings) of lock pins 67 standing up from the top of the middle container 42 at locations corresponding to the fitting holes 56 (Fig. 13) of the upper container 41, and they are integrally formed at those locations. Each of the lock pins 67 preferably has a plurality of split structures. That is, as shown in Fig. 24A, it is constructed of a pair of symmetrical standing segments 68 that stand with a groove-like spacing 48 between them. A widening-diameter section 69 having a widening dimension is formed at the top of each standing segment 68, and therefore the apex of the standing segment 68 has the approximate shape of an abacus bead. This creates an elastic property allowing expansion and contraction in the radial direction at the top of the abacus bead-shaped apex.
In the examples shown in Figs. 24A and 24B, the standing segment 68 is shown as having a split structure, but there is no limitation to this structure and for example, grooves having prescribed widthwise dimensions may be provided every 90 degrees along the longitudinal direction, or the grooves may be provided at other angles such as every 60 degrees or every 120 degrees. It is important, however, for the grooves to be formed at equivalent angles, so that the elastic property is not exhibited in an asymmetrical manner. The lock pin 67 has a length such that it does not protrude from the surface of the upper container 41, as shown in Fig. 17, when the lock pin is fitted in the fitting hole 56 of the upper container 41 (Fig. 11 and Fig. 17). In order to facilitate insertion of the lock pin 67 into the fitting hole 56 of the upper container 41, the lower section 58 of the fitting hole 56 has a gently narrowing diameter from bottom to top, as shown in Fig. 11, so that pressing the lock pin 67 into the fitting hole 56 causes the widening-diameter section 69 of the lock pin 67 to gradually reduce in diameter in an elastic manner so that it is easily pressed into the fitting hole 56.
When the widening-diameter section 69 of the lock pin 67 reaches the step 59 of the fitting hole 56, the widening-diameter section 69 automatically widens by elastic force, allowing it to easily engage with the step 59. In the examples shown in Fig. 11 and Fig. 17, the lock pin 67 cannot be separated from the fitting hole 56 since it is in the "fixed" state; however, when a problem has been found in the filter after assembly, for example, it may be necessary to remove and exchange the deficient filter. Thus, the widths of the longitudinal grooves formed in the widening-diameter section 69 of the lock pin 67 can be appropriately adjusted so that it can be separated from the fitting hole 56 when necessary.

A plurality (four in the example of Fig. 9) of roughly elliptical contact rings 70 facing downward are formed around the perimeter of each guide wall 64 suspended from the middle container 42, so as to surround each guide wall 64. The arrangements of the contact rings 70 are shown in Figs. 9, 23 and 20 which shows the back side of the middle container 42. The contact rings 70 are in contact with the top of the auxiliary packing 47 described below, and function to accurately position the auxiliary packing 47 at the prescribed location when the auxiliary packing 47 is pressed from the top. The contact rings 70 are formed in a roughly elliptical manner because the openings 94 of the auxiliary packing 47 are also roughly elliptical. The plurality of contact rings 70 are not limited to a number of four, similar to the contact rings 55, 66 mentioned above.

Two packing members are sandwiched between the upper container 41 and the middle container 42. As shown in Fig. 9, these are the top packing 44 whose top side is held by the upper container 41 and the bottom packing 45 whose bottom side is held by the middle container 42. The packings are formed to essentially the same surface area and thickness with essentially the same material which may be, for example, a soft material such as silicon, and when the packings 44, 45 are pressed and held against the upper container 41 and the middle container 42, respectively, the contact rings 55, 66 formed in the upper container 41 and the middle container 42, respectively, sink into the packings of soft materials, thus preventing slippage of the packings 44, 45 while also preventing displacement between the packings. As mentioned above, the outer perimeters of the packings 44, 45 are positioned and held in contact with the inside of the external vertical wall 52 of the upper container 41, for positioning of the packings.

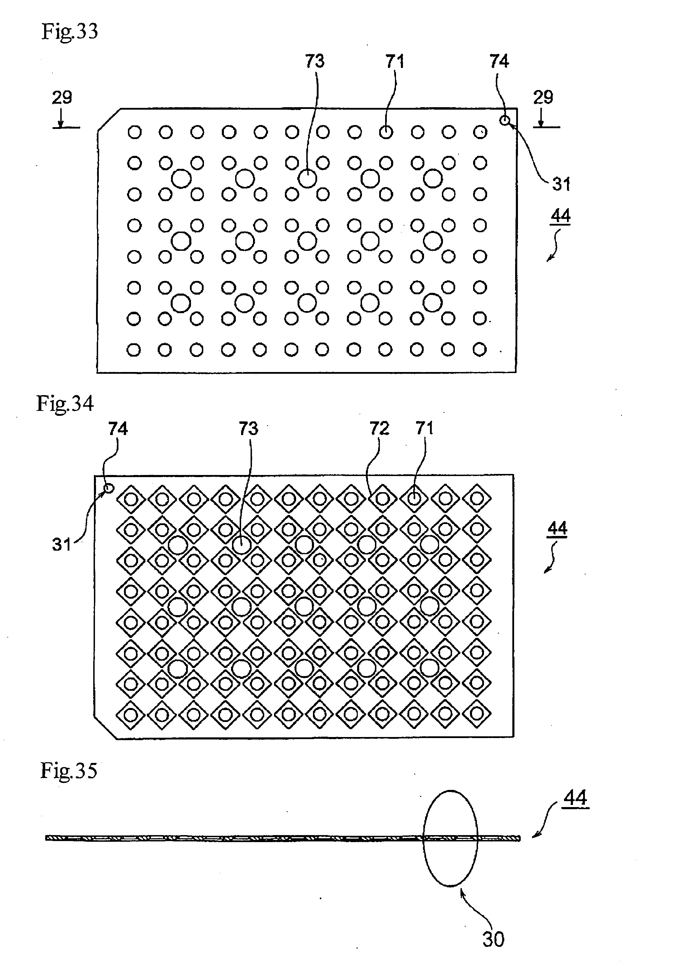

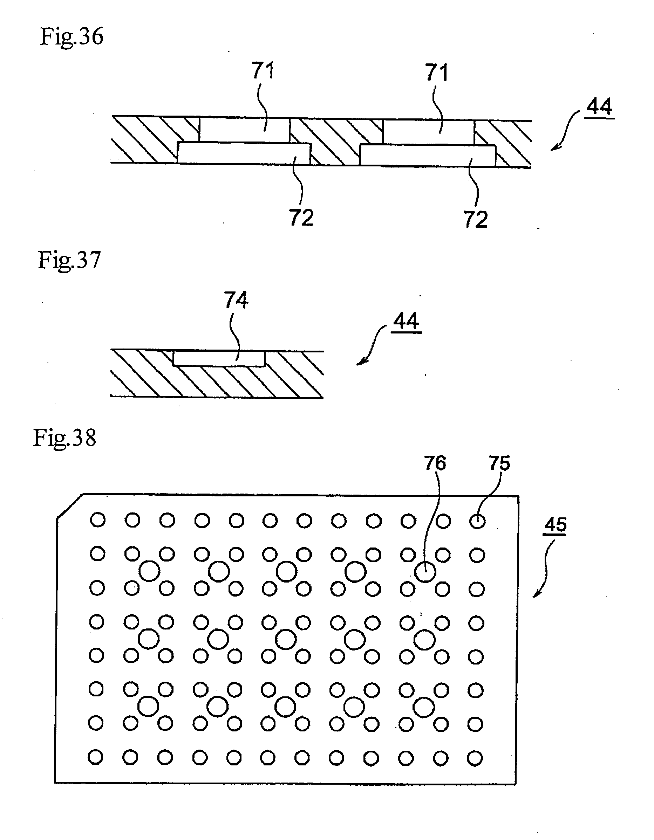

The packings 44, 45 will now be explained.
The top packing 44 shown in Fig. 33 to Fig. 37 has a plurality (96 in the examples of the drawings) of circular openings 71 formed at positions corresponding to the openings 50 of the upper container 41 (Fig. 33). The openings 71 are formed through the packing 44 itself. A rectangular step 72 is provided on the rear side of the packing 44 around the periphery of each opening 71 (Fig. 34 and Fig. 36). The shape and depth of the step 72 essentially match the shape and thickness of the filter 46, described hereunder. Since rectangular filters are assumed for the example in the drawings, the step 72 is also rectangular, but when circular filters are used the step 72 will be a circular shape with a wider diameter than the openings 71. The diameter of the opening 71 is also preferably essentially equal to the diameter defined by the bottom end of the conical-shaped wall 51 formed in the upper container 41. This will prevent leakage of the substance to be tested and allow examination to be performed without waste.
A plurality (15 in the examples of the drawings) of holes 73 are formed through the packing, between the openings 50 of the packing 44, having somewhat larger dimensions than the dimensions of the openings 50. The holes 73 receive the lower sections 58 of the fitting holes 56 formed in the upper container 41 (Fig. 17). Also, indentations 74 for positioning are provided at corners of the top packing 44. The indentations 74 are formed as shallow recesses at prescribed positions, as shown in Fig. 37. In this example one is provided at different corners of the top side (Fig. 33) and bottom side (Fig. 34) of the packing 44, but there is no limitation to this construction. One indentation may be provided at each corner on the top and bottom sides, for a total of eight, or only one may be provided on only one side. This is because the indentations 74 have the function of defining reference points when the filters 46 are mounted on the openings 71 of the packing 44, and therefore several reference points may be necessary depending on the filter mounting mechanism.

The bottom packing 45 shown in Fig. 38 also has a plurality (96 in the examples of the drawing) of circular openings 75 formed at positions corresponding to the openings 50 of the upper container 41, similarly to the top packing 44. The diameter of the opening 75 is also preferably essentially equal to the diameter defined by the bottom end of the conical-shaped wall 51 formed in the upper container 41. A plurality (15 in the example of the drawing) of holes 76 are formed through the packing, also between the openings 75 of the bottom packing 45, having somewhat larger dimensions than the dimensions of the openings 75. The holes 76 receive the lower sections 58 of the fitting holes 56 formed in the upper container 41 (Fig. 17).
The bottom packing 45 has the same dimensions and shape as the top packing 44 but does not have the steps 72 that are formed in the top packing 44, and since the openings 75 and holes 76 run from the top side through to the rear side, the bottom packing 45 has approximately the same shape on its top and rear sides. The bottom packing 45 also has essentially the same thickness as the top packing 44. In the examples shown in the drawing, the steps 72 for holding the filter 46 are formed in the top packing 44 for easier workability during assembly, but the steps may instead be formed in the bottom packing 45 and the filters 46 housed therein. Also, steps having dimensions with approximately half the thickness of the outer perimeter of the filters 46 may be formed in the top packing and bottom packing, with the filters 46 clamped and anchored at the prescribed positions by both packings.

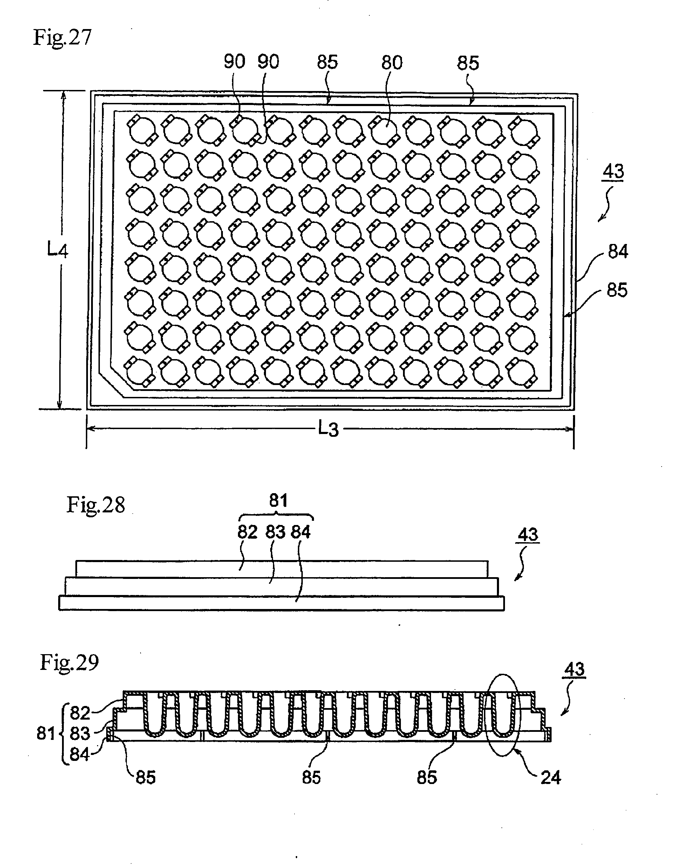

The lower container 43 will now be explained with reference to Figs. 26 to Fig. 32. A plurality (96 in the examples of the drawings) of reservoirs 80 are formed in the lower container 43, suspending roughly perpendicular in an integral manner from the surface of the lower container 43, at locations corresponding to the openings 50 of the upper container 41 (Figs. 9, 12 and 29). The reservoirs 80 serve the function of receiving and housing only the filtered test sample after the substance to be tested supplied through the openings 50 of the upper container 41 has been filtered by the filter 46. Each reservoir 80 has a large enough volume to house the necessary amount of test sample. A peripheral rib 81 is integrally formed in the outer peripheral section of the lower container 43, extending downward from the reservoir 80. The peripheral rib 81 comprises a first suspended section 82 extending out and downward from the surface of the lower container 43, a second suspended section 83 extending further downward from the step that extends roughly horizontally from the lower end of the first suspended section 82, and a third suspended section 84 that extends further downward from the step extending roughly horizontally from the lower end of the second suspended section 83.
The peripheral rib 81 has the function of allowing stacking to be carried out in a stable manner when it is attempted to stack a plurality of microplates 40 according to the invention on one another. Specifically, as shown in Fig. 27, the widthwise inner dimension L3 and the lengthwise inner dimension L4 of the third suspended section 84 are approximately equal to the widthwise dimension L5 and lengthwise dimension L5, respectively, of the upper container 41 shown in Fig. 13, and in order to ensure stable stacking, a plurality of stack ribs 85 (10 in the example of the drawing) are integrally formed with the inner wall of the third suspended section 84, as shown in Fig. 27. Upon stacking, the stack ribs 85 engage with the outer periphery of the upper containers 41, thus maintaining a stable stacked condition. Also, the external dimensions of the first suspended section 82 are slightly smaller than the inner dimensions of the retainer wall section 62 of the middle container 42, thus providing a relationship that allows essentially firm fitting, as shown in Fig. 9.

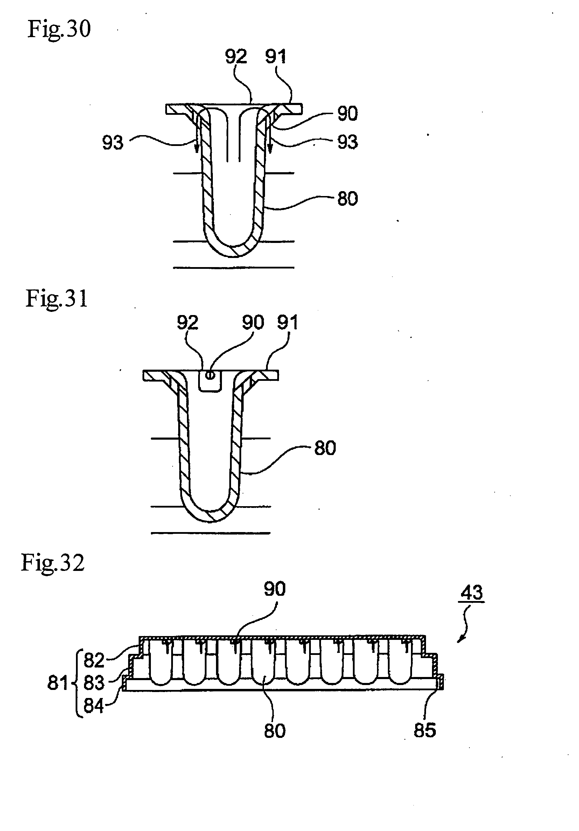

As mentioned above, a plurality of reservoirs 80 are integrally formed in the lower container 43. When these reservoirs 80 are viewed from above, as shown in Fig. 26, a pair of vents 90 are seen to be formed at opposite positions in the diameter direction of the reservoir 80. As shown in Fig. 30, these vents 90 are formed at the widening slant 92 where the neck of each reservoir 80 meets the top side 91 of the lower container 43, extending perpendicularly with respect to the top side 91. The upper edge of the hole of each vent 90 therefore communicates with the widening slant 92 and opens into it. Consequently, when a negative pressure is applied from outside the reservoir 80, the interior of the reservoir 80 is brought to a reduced pressure state from the top of the reservoir 80 through the vents 90, as indicated by the curved arrows 93. Fig. 31 shows the widening slant 92 and the vents 90 that open into it at the top. Fig. 32 shows vents 90 that are open to the outside of the reservoirs 80. Fig. 27 shows a view from below the lower container 43.
The reservoirs 80 are represented as larger than the reservoirs 80 shown in Fig. 26 because Fig. 26 shows the inner sections whereas Fig. 27 shows the outer sections of the reservoirs 80. In Fig. 27, the sections of the reservoirs 80 that protrude radially outward from the outer diameter sections on opposite sites in the diameter direction represent the widening slants 92 and the vents 90 formed in them, as viewed from below. The shapes of the reservoirs 80 in Fig. 30 and Fig. 31 are slightly different. Specifically, the reservoirs in Fig. 30 are formed narrowly overall, while the reservoirs in Fig. 31 are formed wider overall than in Fig. 24. This is because, as shown by the cross-sectional views of Fig. 26, in Fig. 30 the cross-section is along the direction connecting the pair of vents so that the reservoir forms a widening slant, thus being depressed in the slant direction, whereas in Fig. 31 the cross-section is along the direction perpendicular to the direction connecting the pair of vents, so that there is no effect of the widening slant forming the reservoir. This relationship is also the same in Fig. 12 and Fig. 9.

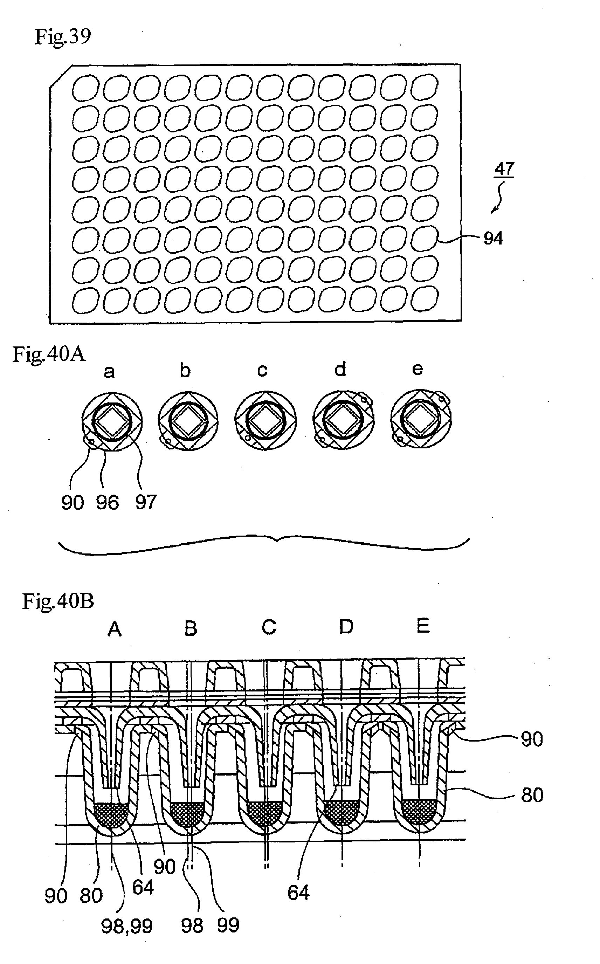

An auxiliary packing 47 as shown in Fig. 39 is mounted between the middle container 42 and the lower container 43. The auxiliary packing 47 is constructed of essentially the same material as the top packing 44 and the bottom packing 45, and its thickness and area is also about the same as these packings, while also having the same number of openings 94 formed therein. However, the shapes of the openings 94 are approximately elliptical, as the shapes of the contact rings 70 provided on the rear side of the middle container 42. The sizes, however, are somewhat smaller than the contact rings 70. This is because, as seen in Fig. 9, the openings 94 are formed more inward than the positions of the contact rings 70. The openings 94 have approximately elliptical shapes in order not to block the vents 90 formed around the reservoirs 80 in the middle container 42. The openings 94 are formed from the top through to the rear side, and therefore have roughly the same shapes on the top and rear sides of the auxiliary packing 47.

The filters 46 (Fig. 9) function to separate out only the necessary elements from the substance to be tested provided from the upper container 41 to openings 50 and send them to the reservoirs 80 of the lower container 43, and they will normally be made of filtration materials with homogeneous through-holes to precisely collect the specific substances of interest. They will usuallybe formed by etching of a silicon wafer. The diameters of the holes of the filters are determined according to the size of the specific substance of interest. In order to shorten the filtering time, the filters 46 are thin-films with very small thicknesses. Since they will therefore be very prone to damage, the utmost care is necessary for their handling. Therefore, the sections where they are set on the microplate in the example of the drawing, i.e. the sections held in the steps 72 formed in the top packing 44 of Fig. 36, for this example, are thicker, as shown in Fig. 9 and Fig. 12. Also, the filter 46 has a slanted section 49 that is gradually slanted from the thick perimeter section held at the steps 72 toward the thin-film section at the center. In the example shown in the drawing, the filter 46 has a rectangular shape while the steps 72 of the top packing 44 which receive the filters are also rectangular, but there is no limitation to this shape, as mentioned above.

Assembly of the filter-equipped microplate of the invention is accomplished by first setting the upper container 41 in an inverted state. The top packing 44 is then placed on the inverted upper container 41. At this time care must be paid so that the steps 72 of the top packing 44 are directed upward. The filters 46 are then set on the steps 72 of the top packing 44. Here, the filters 46 are placed in the opposite position (inverted) from the position in which they are used during operation. The filters 46 are extremely thin overall, and the circular center sections placed at the openings 71 in Fig. 34 are particularly fragile, so that the utmost care is required for their handling. The sections of the filters 46 from the thin sections at the center positioned over the openings 71 to the somewhat thicker periphery held at the steps 72 are connected to the slanted sections 49. The bottom packing 45 is then placed on top of the top packing 44 and the filters 46. When steps are formed on the bottom packing to hold the filters 46, the bottom packing already having the filters 46 set on the steps is placed on the top packing after the top packing has been set. Thus, the top packing 44 and the bottom packing 45 are appropriately placed inside the external vertical wall 52 of the upper container 41.

As alternative means, the filter-attached top packing 44 may be set on the inverted upper container 41 after the filters 46 have already been mounted on the top packing 44. Thus, indentations 74 are provided at different corners of the top packing 44 in order to permit appropriate control of the position of the top packing 44. Specifically, fine adjustment of the reference position of the packing 44 may be necessary so that filters 46 that are continuously supplied by a filter-mounting mechanism (not shown) are arranged at their proper locations on the top packing 44. In such cases, an appropriate positioning control mechanism such as an indicator interacts with the indentations 74, thus allowing the filters 46 to be consistently and accurately positioned on the top packing 44. After the filter-attached top packing has been set on the upper container 41, the bottom packing 45 is mounted, thus restricting movement of the filters.

The middle container 42 is then set on top of the bottom packing 45. Here, the middle container 42 is positioned with the protrusion 61-containing standing wall 60 facing downward so as to cover the bottom packing 45, and the middle container 42 is pressed into the side of the upper container 41 until the protrusion 61 of the middle container 42 fully fits into the protrusion 53 formed in the external vertical wall 52 of the upper container 41, thus confirming that the middle container 42 and upper container 41 have achieved a reliable fit at the outer perimeter. The 15 lock pins 67 formed in the middle container 42 are then inserted into the fitting holes 56 of the upper container 41. The diameter dimensions of the lock pins 67 are larger than the diameter dimensions of the lower sections 58 of the fitting holes 56. However, forcible pressing of the lock pins 67 into the gradually narrowing lower section 58 causes the abacus bead-shaped tops of the lock pins 67 to contract toward the center by the space with the gaps 48, thus allowing the lock pins 67 to be easily fitted into the lower section 58. Further pressing of the lock pins 67 into the fitting holes 56 causes the lock pins 67 to reach the upper sections 57 that have wider dimensions. The tops of the lock pins 67 that have been contracted up to this point are thus restored to their normal diameter states.
Consequently, the lock pins 67 are supported at the steps 59 of the fitting holes 56 so that their exit is prevented. It is, therefore, necessary to confirmthat the lockpins 67 have reliably engaged with the steps 59. This can be easily confirmed by up-down movement of the lock pins in the axial direction, and by the sound of the lock pins fitting into the steps.

When the protrusion 61 and the lock pins 67 provided in the standing wall 60 around the perimeter of the middle container 42 have been fully snapped into appropriate places, assembly of the upper container 41, the middle container 42, the top packing 44, the bottom packing 45 and the filters 46 is complete. In this state, the contact rings 55 provided under the conical-shaped walls 51 of the upper container 41 are pressed against the top packing 44, while the contact rings 66 provided around the openings of the middle container 42 are pressed against the bottom packing 45. The packings 44, 45 are both composed of a soft material such as silicon, and therefore become pressure welded together. Slippage of the filters 46 is thus completely prevented. Also, the upper container 41 and the middle container 42 are bonded into a firm fit by the undercut fit of the protrusions 53, 61 and by the ratchet fit between the fitting hole 56 and lock pin 67.

Next, the auxiliary packing 47 is properly set in the region defined by the retainer wall section 62 extending upward from the inverted middle container 42. At this time, the auxiliary packing 47 is held at the desired location by the contact rings (4 in the example of the drawing) 70 provided around the openings 63 of the middle container 42. Finally, the lower container 43 is attached. The lower container 43 is inverted and mounted onto the middle container 42 which is likewise inverted and has its retainer wall section 62 facing upward. Assembly results in the outer wall of the first suspended section 82 provided in the lower container 43 being placed in close contact with the inside of the retainer wall section 62 provided on the middle container 42, and the assembly operation is thus complete. Finally, the completely assembled filter-equipped microplate 40 is returned to its usable position, as shown in Fig. 8.
Here, the placement is such that the funnel-shaped guide walls 64 formed on the middle container 42 are fitted in a freely movable manner in the reservoirs 80 of the lower container 43, as shown in Fig. 9. Upon completion of the assembly, a plurality of microplates 40 may be stacked for storage, and when they are stacked, the flange 54 formed in the upper container 41 of the bottom microplate is set against the stack rib 85 on the inside of the third suspended section 84 of the upper microplate, thus allowing secure holding of the microplates. Each microplate is, therefore, stacked in order in a step-like manner, in a stable posture. In order to allow mass production of the microplate of the invention, positioning marks may be provided on each part for reference by the assembly device to determine the location of each part and the proper positioning of each part. The assembly operation may then be automated and mass production becomes possible. Since positioning of the filters 46, which are prone to damage against the upper container 41, is important during the operation for assembly of the microplate of the invention, it is important to provide indentations 74 for positioning at the top and/or bottom of the top packing 44, so as to allow proper positioning of the top packing 44 that has the filter-receiving steps 72 that receive each of the filters 46.

A method of using the filter-equipped microplate 40 of Example 2 according to the invention will now be described. As shown in Fig. 8, one filter-equipped microplate 40 is placed in a prescribed horizontal position. The substance to be tested is then supplied to the openings 50 of the upper container 41 using an appropriate tool such as a pipette. In the microplate 40 shown here, the number of specimens that can be simultaneously supplied as test samples is 96, i.e. the number of openings 50. Referring to Fig. 9 which shows an enlarged view of the cross-section of an opening 50, the substance to be tested supplied into the opening 50 defined by the conical-shaped wall 51 of the upper container 41 is dropped onto the thin filtration surface at the center of the filter 46 which is held against the step 72 of the top packing 44 and sandwiched by the bottom packing 45. Only the portion of the test sample having the prescribed properties can pass through the filter 46. The selectively separated test sample is guided by the funnel-shaped guide wall 64 of the middle container 42 and is retained in the reservoir 80 of the lower container 43. It is necessary at this time to take care that the amount of test sample retained in the reservoir 80 is an amount that fits within the region indicated by the dimension L7, from the bottom section of the reservoir 80 to the downward opening 65 at the lower end of the guide wall 64.
Specifically, the amount of sample may be at most about 30 cubic millimeters.

After a fixed amount of the test sample has been retained in the reservoir 80, only the lower container 43 of the microplate 40 is gently separated from the middle container 42. This separating procedure allows easy separation by gripping the second suspended section 83 of the lower container 43 and the standing wall 60 of the middle container 42, and detaching in the vertical direction. While taking care that the test sample retained in the reservoir 80 of the separated lower container does not spill out, the test sample is then carried to an examining table where detailed examination of the sample begins.

Depending on the mesh dimensions of the filter 46 used for the invention, the substance to be tested may not easily pass through the filter 46, and this may predict risk that the substance to be tested may undergo physical changes due to contact with air, for example, during this time. A negative pressure is, therefore, produced in the reservoir 80 of the lower container 43 in the microplate 40 of the invention in order to allow the substance to be tested supplied to the opening 50 to rapidly pass through the filter 46 so that the filtering procedure can be accomplished as rapidly as possible for the substance to be tested, and for this purpose means are provided to forcibly move the substance to be tested through the filter into the reservoir 80. Specifically, the area surrounding the reservoir 80 of the filter-equipped microplate 40 of the invention is reduced in pressure by known pressure reducing means such as a pump. As a result, the air inside the reservoir 80 is evacuated from the reservoir 80 through the pair of vents 90, as indicated by numeral 93 in Fig. 30, thus reducing the pressure in the reservoir 80. Since the interior of the reservoir 80 is at negative pressure, the air in the opening 63 of the middle container 42, which is directly below the filter 46, is drawn out of the reservoir 80 as indicated by numeral 95 in Fig. 9, thus producing a negative pressure in the opening 63. Thus, the substance to be tested supplied to the opening 50 of the upper container 41 is rapidly drawn downward through the filter 46. As a result, the substance to be tested is forcibly moved through the filter 46 into the reservoir 80. As mentioned above, care must be taken at this time that the liquid level of the substance retained in the reservoir 80 does not rise above the dimension L7. In other words, a space must remain between the liquid level of the sample and the downward opening 65 of the guide wall 64. If no space is present, the liquid will be drawn directly into the reduced pressure apparatus, making it impossible to achieve the original purpose.

Fig. 40A and Fig. 40B show the relationship between the number of vents 90 for evacuation of air in the reservoir 80 to the outside of the reservoir 80, the positions of the vents 90 and the positions of the guide walls 64 in the reservoirs 80. In Fig. 40A and Fig. 40B, A(a), B(b) and C(c) represent examples with one vent 90, and D(d) and E(e) represent examples with two vents 90 provided opposite each other in the diameter direction. Also, a, b and d are examples wherein the vents 90 are provided on a circumscribed circle 96 around the corners of the square filter 46, while c and e are examples wherein they are formed on the inside of the circumscribed circle 96.
More specifically, the example A(a) is an example
wherein the lengthwise axial line 98 of the reservoir 80 and the lengthwise axial line 99 of the guide wall 64 are coaxial, wherein the space in which the air in the reservoir 80 moves during pressure reduction is approximately equal around the guide wall 64. The example B(b) has the guide wall 64 nearer the side opposite the side in which the vent 90 is formed. The example C(c) has one vent 90 formed near the reservoir 80 and, as in the example B(b), has the guide wall 64 nearer the side opposite the side in which the vent 90 is formed. The example D(d) is similar to the example A(a), but it has a pair of vents 90 opposite each other in the diameter direction. Finally, the example E(e), like the examples A(a) and D(d), has the lengthwise axial line 98 of the reservoir 80 matching the lengthwise axial line 99 of the guide wall 64, but the pair of vents 90 opposite each other in the diameter direction are provided near the reservoir 80.
According to experimentation by the present inventors, it has been found that a closer position of the vents 90 to the reservoir 80 minimizes pressure reduction loss. However, significant variation was found to occur depending on the strength of pressure reduction and the liquid volume in the reservoir 80. In examples a-e of Fig. 40A and Fig. 40B, the circle 97 inside the circumscribed circle 96 represents the circumscribed circle around the slanted section 49 of the filter 46. Also, in A-E of Fig. 40A and Fig. 40B, the hatched area inside the reservoir 80 represents sample and the amount is about 30 cubic millimeters. The examples shown in the drawings include cases with only one or two vents 90, but more vents can be provided, and it was confirmed that the same excellent effect is exhibited even with 4 or 6 vents.

### [Example 3]

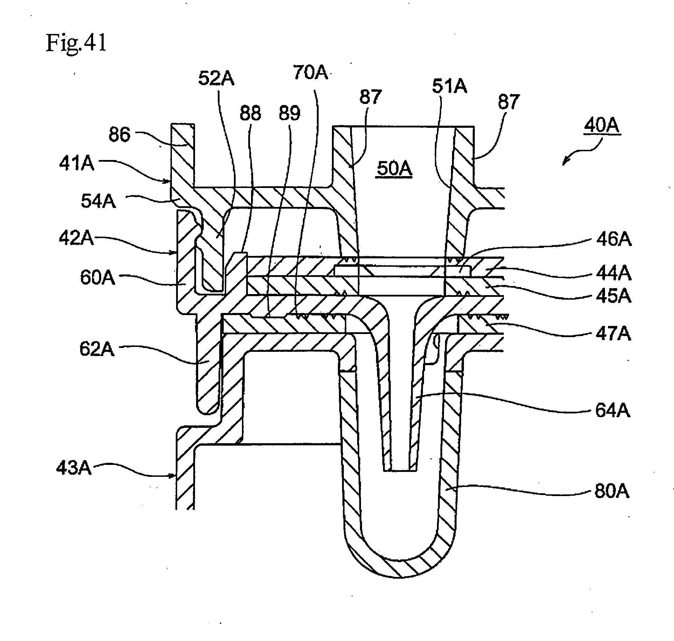

Example 3 according to the invention will now be explained with reference to Fig. 41. Example 3 shown in Fig. 41 is similar to Example 2 described above, and therefore only the aspects differing from that example will be explained here. In Fig. 41, the elements and sections similar to the example described above are indicated by the same numbers, with the letter "A". As clearly seen by the example shown in Fig. 41, the elements composing the Fig. 41 have slightly different shapes from the elements shown in the previous drawings, particularly Fig. 9, but the basic structures of the respective elements are essentially identical to those of the previous examples and merely constitute modifications that are very easily understood by one skilled in the art, and therefore detailed illustrations of each of the constituent elements of Example 3 are omitted.

In a filter-equipped microplate 40A shown in Fig. 41, a flange standing section 86 rises vertically upward from a flange 54A at the periphery of the upper container 41A, preferably in an integral manner with the upper container 41A. Similarly, an open standing section 87 rises upward from the periphery of each opening 50A, preferably in an integral manner with the upper container 41A. Preferably, the height of the flange standing section 86 and the height of the open standing section 87 are essentially identical, or 86 is slightly higher. Whenaplurality of microplates 40A are stacked together during transport or at other times, this allows the microplates 40A to be stacked in a stable manner without slipping to one side and without shaking.
This full set height of the microplates 40A is increased due to the provision of the flange standing section 86 and the open standing sections 87, also substantially increasing the height dimension L1 of the opening 50 shown in Fig. 3, thereby facilitating handling of the microplates 40A. In addition, the areas of the openings 50A are widened and the sample can be more easily supplied to the openings 50A. In the example shown in Fig. 9, the dimension L2 of the external vertical wall 52 is somewhat larger than the dimension L1 of the conical-shaped wall 51, and as a result the external vertical wall 52 can surround the packings 44, 45 positioned below the conical-shaped wall 51, in the region defined by the vertical wall 52; however, in the example shown in Fig. 41, this surrounding of the packings 44A, 45A is achieved by the inner standing wall 88 provided by the middle container 42A. At the bottom end of the conical-shaped wall 51A there is also provided a contact ring, indicated by 55 in Fig. 9.

The middle container 42A, which is positioned under and against the upper container 41A and which cooperates with the upper container 41A to sandwich the top packing 44A and the bottom packing 45A, has the function of connecting together the upper container 41A and the lower container 43A. The middle container 42A, as shown in Fig. 41, has a standing wall 60A that rises in an integral fashion from its outer periphery, roughly in the perpendicular direction toward the upper container 41A. The standing wall 60A rises to a position that surrounds the outside of the external vertical wall 52A of the upper container 41A. Also, the middle container 42A has, preferably around its perimeter, an inner standing wall 88 rising in an integral fashion to a position bordering the inner surface of the external vertical wall 52A of the upper container 41A. Thus, the external vertical wall 52A of the upper container 41A fits firmly in the groove defined by the inner wall surface of the standing wall 60A and the outer wall surface of the inner standing wall 88. A stronger fit is, therefore, achieved between the upper container 41A and the middle container 42A. The inner standing wall 88 is constructed to a lower height than the standing wall 60A, and a small angular notch is formed in the outer upper section of the inner standing wall 88 to facilitate fitting of the upper container 41A into the groove. The inner standing wall 88 also has the function of matching up the outer perimeters of the top packing 44A and the bottom packing 45A. Consequently, the height of the inner side of the inner standing wall 88 is set to a sufficient height to hold the packings 44A and 45A.
In the example shown in Fig. 9 the outer perimeters of the packings 44, 45 are held against the inner surface of the external vertical wall 52 of the upper container 41, but the third example shown in Fig. 41 differs in that they are held against the inner surface of the inner standing wall 88 of the middle container 42A. As a result, when the top packing andbottompacking are assembled in the example shown in Fig. 9, the packings 44, 45 are assembled with the upper container 41 in an inverted state and then the middle container 42 is assembled with the upper container 41, whereas in Example 3 shown in Fig. 41, the middle container 42A may be assembled with the inverted upper container 41A after the packings 44A, 45A have been assembled with the middle container 42A. Also, the packings 44A, 45A used in Example 3 are formed to smaller sizes than the packings 44, 45 used in Example 2, by an amount equal to the thickness of the inner standing wall 88.

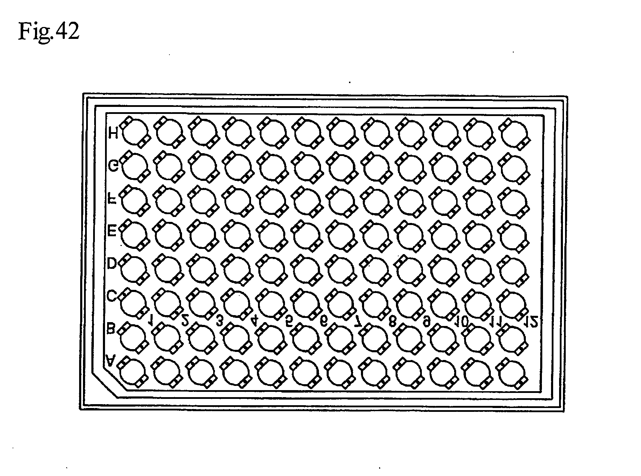

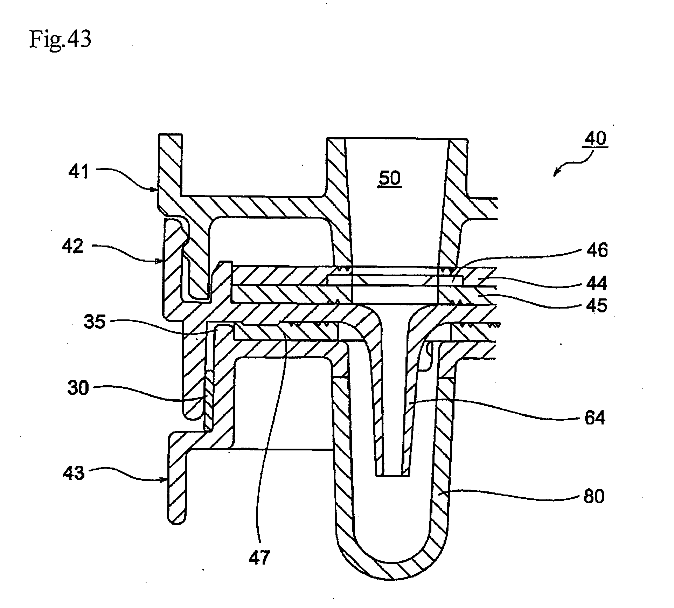

A retainer wall section 62A is integrally formed near the outer periphery of the middle container 42A, suspending downward therefrom in the direction opposite from the standing wall 60A. The retainer wall section 62A has the function of fitting the middle container 42Awith the lower container 4 3A while holding the auxiliary packing 47A. Also, the middle container 42A has guide walls 64A integrally in the same number as the openings 50A of the upper container 41A and at locations corresponding to the openings 50A. The guide walls 64A have the function of guiding sample that has been supplied to the openings 50A and has been filtered through the filters 46A, into the reservoirs 80A of the lower container 43A. Contact rings 70A are provided to hold the auxiliary packing 47A at a prescribed position between the retainer wall section 62A and the guide wall 64A. This construction is the same as Example 2 shown in Fig. 9. In Example 2 shown in Fig. 41, the middle container 42A also has a downward projecting protrusion 89 between the contact rings 70A and the retainer wall section 62A integrally.
The protrusion 89 is preferably formed outside of all of the contact rings 70A formed around the guide walls 64A, but there is no limitation to such a construction, and a plurality of protrusions may be provided on the inner side of the retainer wall section 62A along the widthwise direction and/or lengthwise direction of the middle container 42A. The protrusions 89 serve to ensure that the auxiliary packing 47A definitely sticks to the lower container 43A when the lower container 43A is removed from the middle container 42A after the sample has been drawn into the reservoir 80A. This can prevent the inconvenience of the auxiliary packing 47A attaching to the middle container 42A and unexpectedly separating from the middle container 42A and contaminating the sample in the reservoir 80A, when the lower container 43A is removed from the middle container 42A. The tips of the contact rings 70A are preferably acute angles since they serve to properly position the auxiliary packing 47A, whereas the tips of the protrusions 89 preferably have flat or rounded cross-sections since they serve to aid separation of the auxiliary packing 47A from the middle container 42A.
The other aspects of the construction described above for Example 3 shown in Fig. 41 are essentially the same as the construction of Example 2 shown in Fig. 7 to Fig. 40B, and the method of use is also the same as in Example 2.
Since a plurality of openings are present according to the invention, number or letter references are provided at locations near the openings of the upper container and/or lower container to identify the locations of each of the openings. Specifically, as shown in Fig. 1, the locations of the openings may be identified by A, B, C, etc. in the longitudinal direction of the upper container surface and by 1, 2, 3, etc. in the transverse direction. Also, as shown in Fig. 42, the lower container may be worked from the back side, for example, so that A, B, C, etc. and 1, 2, 3, etc. can be properly recognized when viewed from above the front side of the upper container.
Also, to prevent rattling when the middle container 42 and the lower container 43 are set or, when a plurality of microplates are stacked together for transport or the like, to allow the microplates to be stacked in a stable manner without slipping to one side and without shaking, a plurality of lower container ribs 30 are provided at points on the side in contact with the middle container 42 of the lower container 43, as shown in Fig. 43. The ribs 30 may be provided at, for example, 3 locations in the lengthwise direction and 2 locations in the widthwise direction of the filter-equipped microplate.
Similarly, a lower container guide 35 is provided along the full top perimeter of the section in contact with the outer periphery of the auxiliary packing 47 of the lower container 43, as shown in Fig. 43, in order to prevent rattling when the lower container 43 and auxiliary packing 47 are set.

### Industrial Applicability

The filter-equipped microplate of the invention can be set in a stable manner without damaging thin, fragile filters, and its construction is such that the microplate itself is inexpensive and the tray retaining test sample can be easily separated after filtering so that sample can be supplied to subsequent steps such as mass spectrometry. Furthermore, since the reservoirs have a large depth and the guide walls through which the sample is supplied into the reservoirs have long lengths, it is possible to prevent mixing of adjacent samples when a large negative pressure is applied to draw out the sample during testing, as well as to prevent reverse flow of the sample as it is drawn out and to prevent the samples from being splashed out of the reservoirs.
Consequently, since the requirements for rapid filtering are securely met, it is possible to accomplish rapid drawing of test samples and thus perform analysis of large volumes of samples in a shorter time than has hitherto been possible, while the microplate can also be safely used in other fields such as cell tissue culturing and examination of live cultured tissues. In addition, the filtering can be accomplished using a greater negative pressure than hitherto possible, and therefore a highly industrially useful invention is provided that can reliably meet the demands for rapid and accurate sample analysis with large sample volumes.

## Claims

1. A filter-equipped microplate 110 comprising:
an upper container 111 having openings 120 for injection of a substance to be tested;
a top packing 113 and a bottom packing 114 holding filters 115;
a connecting member 112 fitted with the upper container 111, having openings 140 through which a test sample that has passed through the filters 115 runs and clamping the top packing 113 and the bottom packing 114 against the upper container 111; and
a lower container 116 having reservoirs 160 that retain the test sample, the lower container 116 being held in a freely detachable manner with respect to the connecting member 112.

2. A filter-equipped microplate according to claim 1, wherein the upper container 111 has an external vertical wall 124 that extends vertically downward at the outer periphery; the external vertical wall 124 has a protrusion 125 that projects outward; the connecting member 112 has a standing wall 135 at the outer periphery that extends vertically upward, the standing wall 135 having a protrusion 136 that projects inward; and the protrusions 125, 136 are engaged, whereby fitting between the upper container 111 and the connecting member 112 is achieved by said engagement.

3. A filter-equipped microplate according to claim 1 or 2, wherein the upper container 111 has fitting holes 128, each comprising an upper section 129 and a lower section 130, wherein the upper section 129 has a wide diameter hole and the lower section 130 has a narrow diameter hole, and a step 131 is formed between the upper section 129 and the lower section 130; the connecting member 112 has a hollow lock pin 143 that extends upward, the lock pin 143 having a widening-diameter section 144 at the top, which widening-diameter section 144 comprises a plurality of grooves that extend in the axial direction; and pressing the lock pin 143 from the lower section 130 of the upper container 111 toward the fitting hole 128 causes the widening-diameter section 144 of the lock pin 143 to move toward the center and reduce in diameter, while further pressing causes the lock pin 143 to move to the upper section 129 so that the widening-diameter section 144 engages with the step 131 of the upper container 111, whereby fitting between the upper container 111 and the connecting member 112 is achieved.

4. A filter-equipped microplate according to any one of claims 1 to 3, wherein the connecting member 112 has a retainer wall section 137 that extends downward and a positioning pin 145, the retainer wall section 137 consisting of an outer wall 138 and a slanted inner wall 139 and being placed on the outer periphery in such a manner as to surround the connecting member 112, and the positioning pin 145 having a conical shape, with a plurality thereof being provided at the inner section of the connecting member 112; the lower container 116 comprises on an outer periphery thereof a peripheral rib 161 with a slanted section 162 and a step section 163, and a positioning pin receiver 164 that forms a conical shape; and the lower container 116 is in airtight contact with the connecting member 112 by pressure welding of the slanted section 162 against the slanted inner wall 139, while the positioning pin 145 and the positioning pin receiver 164 are loosely fitted across a prescribed spacing.

5. A filter-equipped microplate according to any one of claims 1 to 4, wherein the filter 115 is fabricated by etching of a silicon wafer and comprises a center section with through-holes of equal dimensions and an outer peripheral section surrounding the center section, the outer peripheral section being formed to a greater thickness than the center section.

6. A filter-equipped microplate according to any one of claims 1 to 5, wherein a sealing member such as an O-ring is fitted on either the slanted inner wall 139 of the connecting member 112 or the slanted section 162 of the lower container 116, whereby airtight fitting is achieved between them.

7. A filter-equipped microplate according to claim 4, wherein the connecting member 112 and the lower container 116 are fitted in an airtight manner at the slanted inner wall 139 and the slanted section 162 while being fitted loosely at the other sections, and connection of pressure reducing means to one positioning pin receiver 164 allows negative pressure to be produced below the filter, whereby the filtering time can be shortened.

8. A filter-equipped microplate according to any one of claims 1 to 7, wherein the top packing 113 and the bottom packing 114 form an integral structure.

9. A filter-equipped microplate according to any one of claims 1 to 8, wherein a mark is provided on each filter 115 or a member in contact with the filter 115 to identify the assembly location.

10. A filter-equipped microplate 40, 40A comprising:
an upper container 41, 41A having openings 50, 50A for injection of a substance to be tested;
a top packing44, 44A and a bottom packing 45, 45A holding filters 46, 46A;
a middle container 42, 42A fitted with the upper container 41, 41A , having openings 63 through which a test sample that has passed through the filters 46, 46A runs and clamping the top packing 44, 44A and the bottom packing 45, 45A against the upper container 41, 41A; and
a lower container 43, 43A having reservoirs 80, 80A that retain the test sample, the lower container 43, 43A being held in a freely detachable manner with respect to the middle container 42, 42A,
wherein the middle container 42, 42A has guide walls 64, 64A that suspend down from the openings 63 and provide downward openings 65 to the bottom end, the reservoirs 80, 80A in the lower container 43, 43A house the guide walls 64, 64A, said reservoirs 80, 80A receiving test sample supplied from the downward openings 65 of the guide walls 64, 64A through the filters 46, 46A, and the lower container 43, 43A has a vent 90 at a widening slant 92 at the top, that communicates with the outside of each of the reservoirs 80, 80A.

11. A filter-equipped microplate according to claim 10, wherein a plurality of vents 90 are provided.

12. A filter-equipped microplate according to claim 11, wherein two vents 90 are provided.

13. A filter-equipped microplate according to any one of claims 10 to 12, wherein the bottom ends of the guide walls 64, 64A suspend to a depth of at least half of the reservoirs 80, 80A.

14. A filter-equipped microplate according to any one of claims 10 to 13, wherein an auxiliary packing 47, 47A is mounted between the middle container 42, 42A and the lower container 43, 43A.

15. A filter-equipped microplate according to claim 14, wherein the auxiliary packing 47, 47A is bonded to the lower container 43, 43A by different material molding or insert molding.

16. A filter-equipped microplate according to any one of claims 10 to 15, wherein the upper container 41, 41A has an external vertical wall 52, 52A that extends vertically downward at the outer periphery and the external vertical wall 52, 52A has a protrusion 53 that projects outward; the middle container 42, 42A has a standing wall 60, 60A at the outer periphery that extends vertically upward, the standing wall 60, 60A having a protrusion 61 that projects inward; and the protrusions 53, 61 are engaged, whereby fitting between the upper container 41, 41A and the middle container 42, 42A is achieved by said engagement.

17. A filter-equipped microplate according to any one of claims 10 to 16, wherein the upper container 41, 41A has fitting holes 56, each comprising an upper section 57 and a lower section 58, wherein the upper section 57 has a widening-diameter hole and the lower section 58 forms a hole whose diameter narrows from bottom to top, with a step 59 being formed between the upper section 57 and lower section 58; the middle container 42, 42A has hollow lock pins 67 that extend upward, each lock pin 67 having a widening-diameter section 69 at the top, which widening-diameter section 69 comprises a space 48 that extends in the axial direction; and pressing the lock pin 67 from the lower section 58 of the upper container 41, 41A toward the fitting hole 56 causes the widening-diameter section 69 of the lock pin 67 to move toward the center and reduce in diameter, while further pressing causes the lock pin 67 to move to the upper section 57 so that the widening-diameter section 69 engages with the step 59 of the upper container 41, 41A, whereby fitting between the upper container 41, 41A and the middle container 42, 42A is achieved.

18. A filter-equipped microplate according to any one of claims 10 to 17, wherein the filters 46, 46A are fabricated by etching of a silicon wafer, and each comprises a center section with through-holes of equal dimensions and an outer peripheral section extending from the center section through the slanted section and surrounding the center section, the outer peripheral section being formed to a greater thickness than the center section.

19. A filter-equipped microplate according to any one of claims 10 to 18, wherein each constituent element is composed of a transparent material.

20. A filter-equipped microplate according to any one of claims 10 to 19, wherein the upper container 41A has a flange standing section 86 that extends upward from the outer peripheral section and an open standing section 87 that extends upward continuously from the opening 50A, the flange standing section 86 and the open standing section 87 extending up to essentially the same height.

21. A filter-equipped microplate according to any one of claims 10 to 20, wherein the middle container 42A has a standing wall 60A extending upward from the outer peripheral section and an inner standing wall 88 extending upward from the inside at a prescribed distance from the standing wall 60A, an external vertical wall 52A suspended from the upper container 41A is fitted in the space defined between the standing wall 60A and the inner standing wall 88, and the height of the inner standing wall 88 is lower than the height of the standing wall 60A.

22. A filter-equipped microplate according to claim 21, wherein the inner standing wall 88 holds the outer perimeters of the top packing 44A and the bottom packing 45A.

23. A filter-equipped microplate according to any one of claims 14 to 22, wherein the middle container 42A has a protrusion 89 on the side in contact with the auxiliary packing 47A.

24. A filter-equipped microplate according to any one of claims 1 to 23, wherein a mark is provided at a location near the opening of the upper container 41 and/or lower container 43, to identify and indicate the location of each opening.

25. A filter-equipped microplate according to any one of claims 1 to 24, wherein a lower container rib 30 is provided on the side of the lower container 43 in contact with the middle container 42, to prevent rattling when the middle container 42 and lower container 43 are set.

26. A filter-equipped microplate according to any one of claims 1 to 25, wherein a lower container guide 35 is provided along the full top perimeter of the section of the lower container 43 in contact with the outer periphery of the auxiliary packing 47, to prevent rattling when the lower container 43 and auxiliary packing 47 are set.
